(19) Europäisches Patentamt
European Patent Office
Office européen des brevets

(11) Veröffentlichungsnummer: **0 034 749**
**B1**

# (12) EUROPÄISCHE PATENTSCHRIFT

(45) Veröffentlichungstag der Patentschrift: **06.06.84**

(51) Int. Cl.³: **G 03 C 5/54**, C 07 D 307/83, C 07 D 307/77

(21) Anmeldenummer: **81100920.8**

(22) Anmeldetag: **10.02.81**

(54) **Farbphotographisches Aufzeichnungsmaterial mit nicht diffundierenden Elektronendonor-Vorläuferverbindungen.**

(30) Priorität: **20.02.80 DE 3006268**

(43) Veröffentlichungstag der Anmeldung: **02.09.81 Patentblatt 81/35**

(45) Bekanntmachung des Hinweises auf die Patenterteilung: **06.06.84 Patentblatt 84/23**

(84) Benannte Vertragsstaaten: **BE DE FR GB IT**

(56) Entgegenhaltungen: **Keine**

(73) Patentinhaber: **AGFA-GEVAERT Aktiengesellschaft, D-5090 Leverkusen 1 (DE)**

(72) Erfinder: **Lässig, Wolfgang, Dr., Ziehrerstrasse 6, D-8000 München 71 (DE)**
Erfinder: **Meier, Ernst, Bunsenstrasse 24, D-8000 München 83 (DE)**
Erfinder: **Schleger, Siegfried, Oefelestrasse 19, D-8000 München 90 (DE)**

**EP 0 034 749 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein farbphotographisches Aufzeichnungsmaterial mit mindestens einer lichtempfindlichen Silberhalogenidemulsionsschicht, das nicht diffundierende Elektronendonor-Vorläuferverbindungen enthält, aus denen unter den Bedingungen der alkalischen Entwicklung starke Reduktionsmittel gebildet werden. Insbesondere betrifft die Erfindung ein Aufzeichnungsmaterial, in dem die genannten Elektronendonor-Vorläuferverbindungen in Kombination verwendet werden mit nicht diffundierenden reduzierbaren farbgebenden Verbindungen, die im reduzierten Zustand unter den Bedingungen der alkalischen Entwicklung diffundierende Farbstoffe freisetzen, die als solche oder nach Umwandlung in ihre Metallkomplexe zum Bildaufbau dienen. Des weiteren betrifft die Erfindung neue Verbindungen, die als Elektronendonor-Vorläuferverbindungen geeignet sind.

In der DE-A- 2 809 716 sind farbgebende Verbindungen mit einer elektronenakzeptierenden nukleophilen Vorläufergruppe beschrieben, die im reduzierten Zustand unter den Bedingungen der alkalischen Entwicklung einer intramolekularen nukleophilen Verdrängungsreaktion unterliegen unter Freisetzung eines diffundierenden Farbstoffes. Die Reduktion wird durch sogenannte Elektronendonorverbindungen (ED-Verbindungen) bewirkt, die neben den farbgebenden Verbindungen in den Schichten vorliegen und die bei der Entwicklung bildmäßig oxidiert und damit verbraucht werden.

Die verbleibende bildmäßige Verteilung (positives Restbild) der ED-Verbindungen reagiert mit der farbgebenden Verbindung und löst die bildmäßige Freisetzung diffundierender Farbstoffe aus. Bei den dort beschriebenen ED-Verbindungen handelt es sich beispielsweise um Derivate des Benzisoxazolons, des Hydrochinons, des p-Aminophenols und der Ascorbinsäure. Um die Erzeugung qualitativ hochwertiger Bilder nach dem in der DE-A- 2 809 716 beschriebenen Verfahren zu ermöglichen, müssen die ED-Verbindungen sich nicht nur durch belichtetes Silberhalogenid bzw. durch Silberhalogenidentwickleroxidationsprodukte oxidieren lassen, sondern selbst auch in der Lage sein, die farbgebenden Verbindungen mit elektronenakzeptierender nukleophiler Vorläufergruppe zu reduzieren. Darüber hinaus müssen die jeweiligen Geschwindigkeiten der Oxidations- bzw. Reduktionsreaktion in optimaler Weise so aufeinander eingestellt sein, daß die ED-Verbindung im Zuge der Entwicklung bereits in bemerkenswertem Umfang oxidiert ist, bevor sie ihrerseits die farbgebende Verbindung zu reduzieren vermag. Die bekannten ED-Verbindungen genügen diesen Anforderungen nicht in jeder Hinsicht, was sich beispielsweise in einer unzureichenden Farbdichte und/oder in einem nicht hinnehmbaren Farbschleier der erzeugten Farbübertragungsbilder äußert. Das hohe Reduktionsvermögen geeigneter ED-Verbindungen bedingt zugleich eine hohe Oxidationsempfindlichkeit, was sich bei längerer Lagerung des photographischen Aufzeichnungsmaterials in einem vorzeitigen, nicht bildmäßigen Verbrauch der ED-Verbindungen durch Oxidation und hierdurch verschlechterte Bildeigenschaften des Übertragsbildes äußert, die besonders in einer unzureichenden Farbdichte bestehen. Aus der DE-A- 2 809 716 ist es auch bereits bekannt, ED-Verbindungen nicht als solche, sondern in Form einer weniger oxidationsempfindlichen Vorläuferverbindung zu verwenden, aus der erst unter den Bedingungen der alkalischen Entwicklung die eigentliche ED-Verbindung gebildet wird. Als Beispiele hierfür sind etwa die ED-Verbindungen ED-8, ED-9 und ED-10 der genannten deutschen Offenlegungsschrift anzusehen. Mit den bekannten ED-Vorläuferverbindungen wird aber innerhalb einer akzeptablen Entwicklungszeit nur eine unzureichende Dichte des Farbübertragungsbildes erhalten, was seine Ursache darin haben mag, daß die eigentliche ED-Verbindung entweder nicht rasch genug aus ihrer Vorläuferverbindung entsteht oder ein zu geringes Reduktionsvermögen aufweist.

Der Erfindung liegt die Aufgabe zugrunde, neue oxidationsstabile ED-Vorläuferverbindungen anzugeben, aus denen unter den Bedingungen der alkalischen Entwicklung ED-Verbindungen mit einem hohen Reduktionsvermögen gebildet werden. Sie sollen insbesondere bei dem Farbdiffusionsübertragungsverfahren die Erzeugung von Farbübertragsbildern mit verbesserter Farbdichte ermöglichen, wenn sie in Kombination mit nicht diffundierenden reduzierbaren farbgebenden Verbindungen verwendet werden.

Gegenstand der Erfindung ist ein farbphotographisches Aufzeichnungsmaterial mit mindestens einer lichtempfindlichen Silberhalogenidemulsionsschicht und einer dieser zugeordneten nicht diffundierenden Elektronendonor-Vorläuferverbindung (ED-Vorläuferverbindung), aus der unter alkalischen Entwicklungsbedingungen eine nicht diffundierende ED-Verbindung mit einem hohen Reduktionsvermögen gebildet wird, wobei das Auszeichnungsmaterial als ED-Vorläuferverbindung ein 5gliedriges $\alpha$-Lacton des Hydrochinons enthält, und wobei der Lactonring elektronenanziehende Substituenten enthält, die eine Aufspaltung des Lactonringes bei pH-Werten zwischen 10 und 13 bewirken, wobei die Aufspaltung des Lactonringes in einer heterodispersen Mischung aus einer Lösung von

20 mg der ED-Vorläuferverbindung
5 mg 1-Phenol-pyrazolidon-3 in
5 ml n-Butanol

als feinverteilte organische Phase und einer Lösung von

50 mg Kaliumferricyanid
10 ml Wasser und
5 ml einer Pufferlösung

bei Zimmertemperatur in zwei Messungen ermittelt wird, wobei die erste Messung unter Verwendung einer Pufferlösung bestehend aus

3,092 g Borsäure ($H_3BO_3$)
3,728 g Kaliumchlorid
0,852 g Natriumhydroxid
ad 50 ml Wasser

und die zweite Messung unter Verwendung einer Pufferlösung bestehend aus

3,728 g Kaliumchlorid
1,884 g Natriumhydroxid
ad 50 ml Wasser

erfolgt, wobei bei der ersten Messung Halbwertszeiten von $>10$ min und bei der zweiten Messung Halbwertszeiten von $<10$ min für den oxidativen Abbau der Lactonverbindung erhalten werden und wobei die durch Aufspaltung des Lactonringes entstehende ED-Verbindung ein Redoxpotential von kleiner als +0,255 besitzt, gemessen bei pH 0 gegen eine Normal-Kalomelektrode.

Die in 4-Stellung zu der lactonisierten Hydroxylgruppe vorhandene zweite Hydroxylgruppe des Hydrochinons kann auch durch eine hydrolysierbare Acylgruppe substituiert sein.

Bevorzugte ED-Vorläuferverbindungen sind solche der Benzofuranon-Reihe, die im Lactonring mit elektronenanziehenden Substituenten der folgenden Art substituiert sind: Alkoxycarbonyl, insbesondere mit Alkoxygruppen mit bis zu 5 C-Atomen, Phenoxycarbonyl, carbocyclische oder heterocyclische aromatische Substituenten, insbesondere Phenyl, Diphenyl, Pyridyl, Thienyl, Pyrryl, Indolyl, Imidazolyl, Benzimidazolyl, Oxazolyl oder Benzoxazolyl, ferner Cyan oder Acyl, wobei sich die Acylgruppe vorzugsweise von Arylcarbonsäure ableitet, wie Benzoyl.

Die erfindungsgemäße Aufgabe wird in besonders günstiger Weise durch ein farbphotographisches Aufzeichnungsmaterial gelöst mit mindestens einer lichtempfindlichen Silberhalogenidemulsionsschicht und einer dieser zugeordneten nicht diffundierenden Elektronendonor-Vorläuferverbindung (ED-Vorläuferverbindung), aus der unter alkalischen Entwicklungsbedingungen eine nicht diffundierende Elektronendonorverbindung (ED-Verbindung) gebildet wird und die der folgenden allgemeinen Formel entspricht:

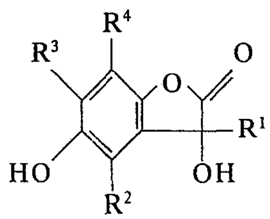

(I)

worin bedeuten:

$R^1$ einen carbocyclischen oder heterocyclischen aromatischen Ring;
$R^2$, $R^3$, $R^4$ gleiche oder verschiedene Substituenten, und zwar Wasserstoff, Alkyl, Alkenyl, Aryl, Alkoxy, Alkylthio oder eine cyclische Aminogruppe, oder
$R^3$ und $R^4$ vervollständigen zusammen einen ankondensierten, insbesondere carbocyclischen Ring

und wobei mindestens einer der Substituenten $R^1$, $R^2$, $R^3$ und $R^4$ einen die Diffusion erschwerenden Ballastrest mit 10–22 C-Atomen enthält.

Die erfindungsgemäßen ED-Vorläuferverbindungen eignen sich für die Verwendung in farbphotographischen Aufzeichnungsmaterialien überall dort, wo es darum geht, starke Reduktionsmittel in verkappter Form in die Schichten einzubringen, sei es als verkapptes Entwicklungsmittel für Silberhalogenid oder als verkapptes Reduktionsmittel zur Verhinderung der unerwünschten Diffusion von Oxidationsprodukten, die aus der Silberhalogenidentwicklung resultieren. Dabei ist es belanglos, welche Art von Farbgebenden Verbindungen zur Farbbilderzeugung zur Anwendung gelangt. Es kann sich hierbei um Farbkuppler handeln oder um farbgebende Verbindungen, die als Folge der Entwicklung diffundierende Farbstoffe in Freiheit setzen. Die bevorzugte Verwendung der erfindungsgemäßen ED-Vorläuferverbindungen ist jedoch in der Kombination mit nicht diffundierenden reduzierbaren farbgebenden Ver-

bindungen zu sehen, die in reduzierter Form unter den Bedingungen der alkalischen Entwicklung diffundierende Farbstoffe in Freiheit setzen.

Ein bevorzugter Gegenstand der Erfindung ist somit ein farbphotographisches Aufzeichnungsmaterial mit mindestens einer lichtempfindlichen Silberhalogenidemulsionsschicht und einer dieser zugeordneten Kombination aus einer nicht diffundierenden reduzierbaren farbgebenden Verbindung, die im reduzierten Zustand unter den alkalischen Entwicklungsbedingungen einen diffundierenden Farbstoff freizusetzen vermag, und einer nicht diffundierenden Elektronendonor-Vorläuferverbindung (ED-Vorläuferverbindung), aus der unter den alkalischen Entwicklungsbedingungen eine nicht diffundierende Elektronendonorverbindung (ED-Verbindung) gebildet wird, die unter den alkalischen Entwicklungsbedingungen die nicht diffundierende farbgebende Verbindung zu reduzieren vermag, dadurch gekennzeichnet, daß das Aufzeichnungsmaterial als ED-Vorläuferverbindung eine Verbindung der allgemeinen Formel I enthält.

Ein weitere Gegenstand sind Verbindungen der allgemeinen Formel I (ED-Vorläuferverbindung).

Der in Formel I durch $R^1$ dargestellte aromatische Rest kann eine carbocyclische Gruppe sein, z. B. eine Phenyl-, Naphthyl- oder Anthracengruppe oder eine 5- oder 6-gliedrige heterocyclische Gruppe mit mindestens einem der Heteroatome N, O, S als Ringglied, z. B. eine Imidazolyl-, Thienyl-, Oxazolyl-, Pyrryl- oder Pyridylgruppe. Die carbocyclischen und heterocyclischen aromatischen Reste können unsubstituiert oder ein- oder mehrfach substituiert sein sowie ankondensierte carbocyclische oder heterocyclische Ringe enthalten, die in diesem Fall nicht aromatisch zu sein brauchen.

Als Substituenten an den durch $R^1$ dargestellten aromatischen Resten sowie gegebenenfalls an hieran ankondensierten Ringen kommen beispielsweise in Frage: Halogen wie Fluor, Chlor, Brom oder Iod, Hydroxy, Sulfo, Sulfamoyl, trifluormethylsulfonyl, Amino, Nitro, Cyano, Carboxy, Carbamoyl, Alkoxycarbonyl, Alkyl, Alkenyl, Cycloalkyl, insbesondere Cyclohexyl oder Cyclopentyl, Aryl, insbesondere Phenyl, oder heterocyclische Gruppen, wobei die zuletzt genannten Gruppen Alkyl bis heterocyclische weitere Substituenten enthalten können, z. B. solche der vorher genannten Art und wobei die genannten Alkyl-, Alkenyl-, Cycloalkyl-, Aryl- oder heterocyclischen Gruppen entweder direkt oder über eines der nachfolgenden zweiwertigen Verbindungsglieder angeknüpft sind: $-O-$, $-S-$, $-SO_2-$, $-SO_2-NR-$, $-NR-SO_2-$, $-NR-CO-$, $-CO-NR-$, $-NR-COO-$, $-O-CO-NR-$, $-NR-CO-NR-$ (R = Wasserstoff oder Alkyl).

Die in den durch $R^2$, $R^3$, $R^4$ dargestellten oder in dem durch $R^1$ dargestellten aromatischen Rest vorhandenen Substituenten enthaltenen Alkyl- oder Alkenylgruppen können gerade oder verzweigt sein und 1—22 C-Atome enthalten.

Eine als Substituent in $R^1$ vorhandene Aminogruppe entspricht der Formel

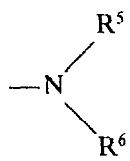

worin $R^5$ und $R^6$ gleich oder verschieden sind und Wasserstoff, Alkyl oder Aryl bedeuten oder die zur Vervollständigung einer 5- oder 6-gliedrigen cyclischen Aminogruppe (Beispiel: Pyrrolidino, Piperidino, Morpholino) erforderlichen Ringglieder.

Eine als $R^2$, $R^3$ oder $R^4$ dargestellte cyclische Aminogruppe entspricht der Formel

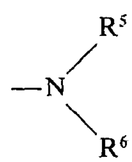

worin $R^5$ und $R^6$ die zur Vervollständigung einer 5- oder 6-gliedrigen cyclischen Aminogruppe (Beispiel: Pyrrolidino, Piperidino, Morpholino) erforderlichen Ringglieder bedeuten.

Erfindungsgemäß ist die Gesamtheit der in einer erfindungsgemäßen ED-Vorläuferverbindung vorhandenen Substituenten so beschaffen, daß die ED-Vorläuferverbindung nicht diffundierend in photographische Schichten eingelagert werden kann. Hierzu enthält beispielsweise mindestens einer der vorhandenen Substituenten, z. B. mindestens einer der $R^1$, $R^2$, $R^3$ und $R^4$, oder ein Substituent an einem durch mindestens zwei der genannten Reste, z. B. durch $R^3$ und $R^4$, vervollständigten Ring einen diffusionsfestmachenden Rest. Eine diffusionsfeste Einlagerung der ED-Vorläuferverbindung ist deshalb besonders erwünscht, weil diese Verbindungen in einer bestimmten Mengenrelation zu den zugeordneten nicht diffundierenden reduzierbaren farbgebenden Verbindungen eingesetzt werden, die sich möglichst auch während einer längeren Lagerung des photographischen Aufzeichnungsmaterials nicht wesentlich ändern soll.

Als diffusionsfestmachende Reste sind solche Reste anzusehen, die es ermöglichen, die erfindungsgemäßen Verbindungen in den üblicherweise bei photographischen Materialien verwendeten hydrophilen Kolloiden diffusionsfest einzulagern. Hierzu sind vorzugsweise organische Reste geeignet, die im allgemeinen geradkettige oder verzweigte aliphatische Gruppen und gegebenenfalls auch carbocyclische oder heterocyclische aromatische Gruppen mit im allgemeinen 10–22 C-Atomen enthalten. Mit dem übrigen Molekülteil sind diese Reste entweder direkt oder indirekt, z. B. über eine der folgenden Gruppen verbunden: $-NHCO-$, $-NHSO_2-$, $-NR-$, wobei R Wasserstoff oder Alkyl bedeutet, $-O-$ oder $-S-$. Zusätzlich kann der diffusionsfestmachende Rest auch wasserlöslichmachende Gruppen enthalten, wie z. B. Sulfogruppen oder Carboxylgruppen, die in bestimmten Fällen auch in anionischer Form vorliegen können. Da die Diffusionseigenschaften von der Molekülgröße der verwendeten Gesamtverbindung abhängen, genügt es in bestimmten Fällen, z. B. wenn das verwendete Gesamtmolekül groß genug ist, oder wenn die ED-Vorläuferverbindungen unter Verwendung sogenannter Ölbildner oder hochsiedender Kupplerlösungsmittel in emulgierter Form in die Schichten eingearbeitet werden, als »diffusionsfestmachende Reste« auch kürzerkettige Reste, z. B. Isoamyl- oder tert.-Butylreste, zu verwenden.

Einige Beispiele für die erfindungsgemäß verwendeten nicht diffundierenden ED-Vorläuferverbindungen sind im folgenden aufgeführt:

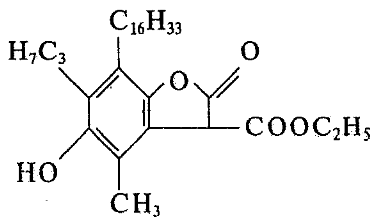

(1)

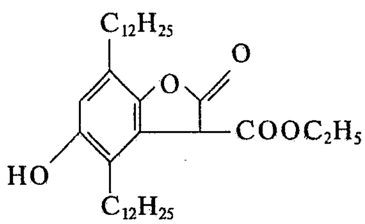

(2)

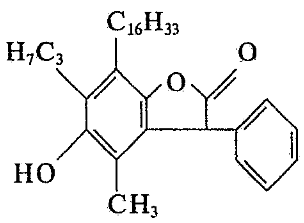

(3)

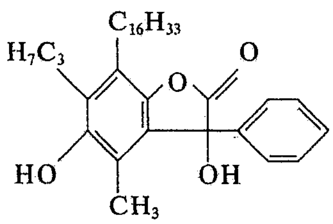

(4)

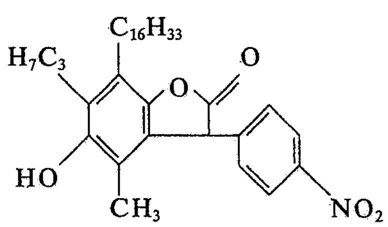

(5)

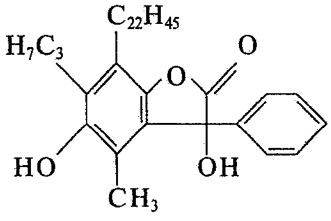

(6)

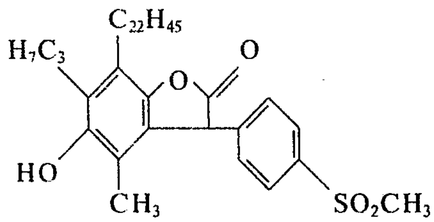

(7)

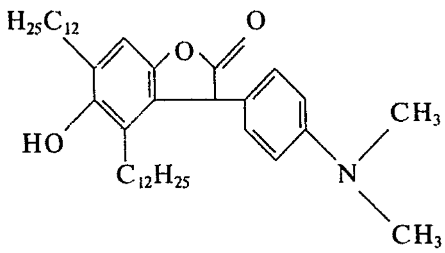

(8)

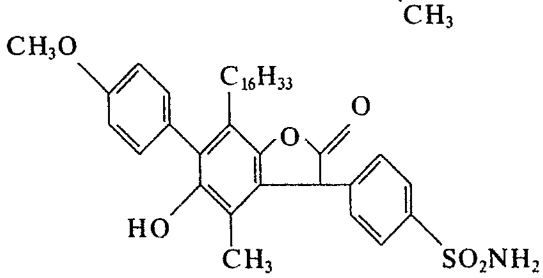

(9)

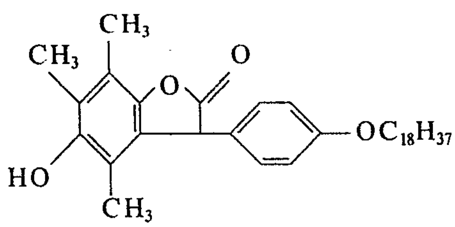

(10)

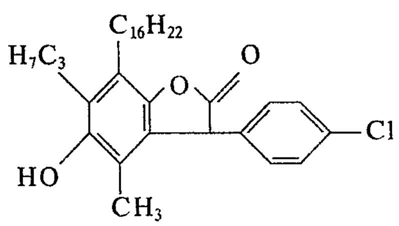

(11)

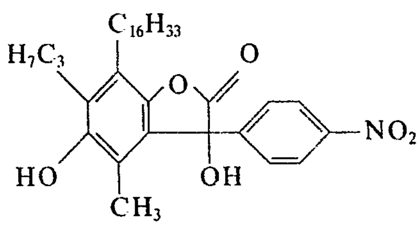

(12)

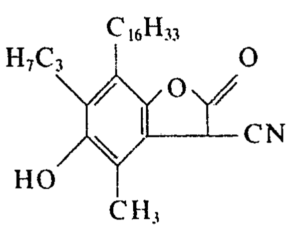

(13)

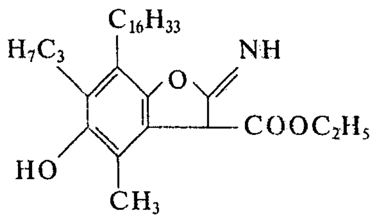

(14)

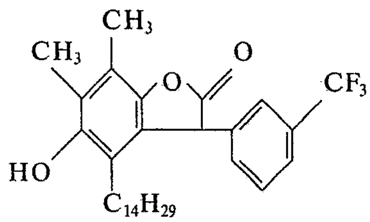

(15)

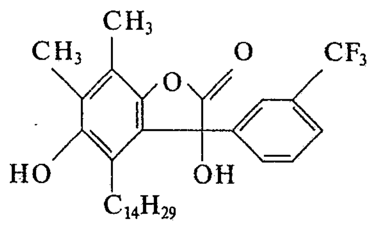

(16)

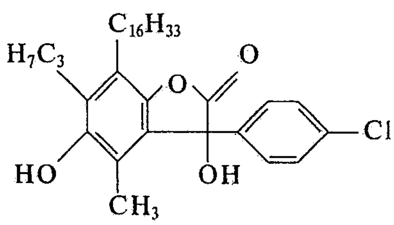

(17)

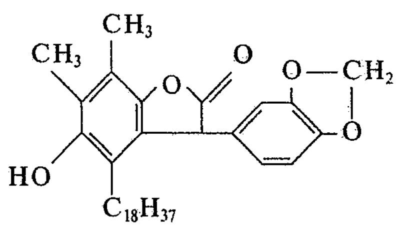

(18)

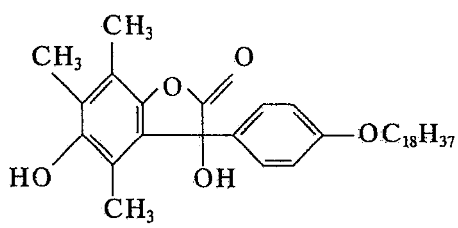

(19)

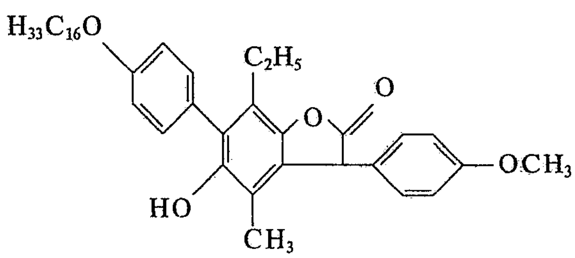

(20)

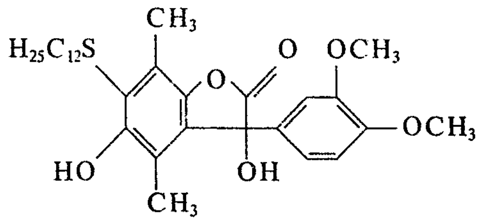

(21)

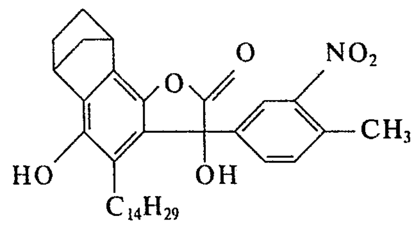

(22)

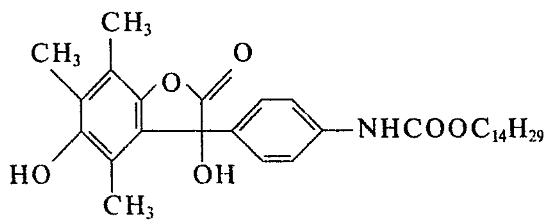

(23)

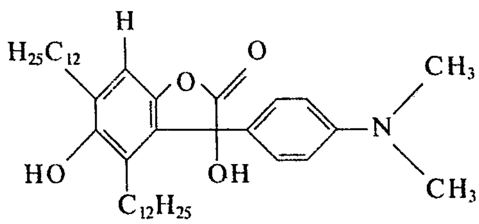

(24)

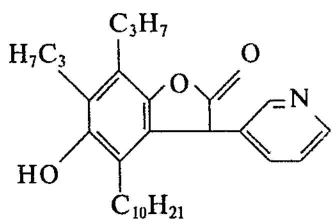

(25)

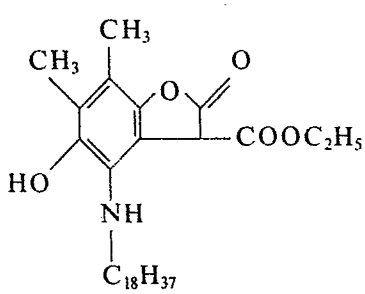

(26)

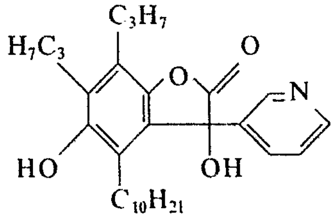

(27)

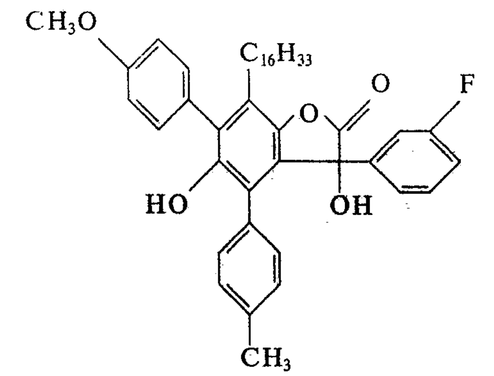

(28)

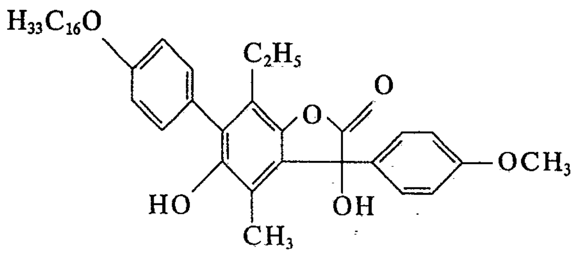

(29)

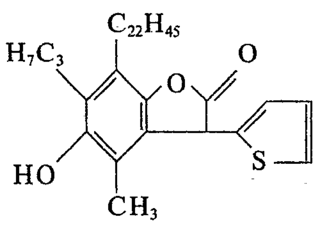

(30)

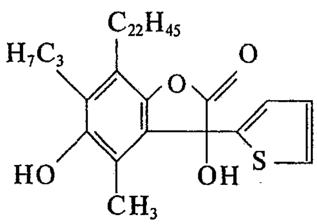

(31)

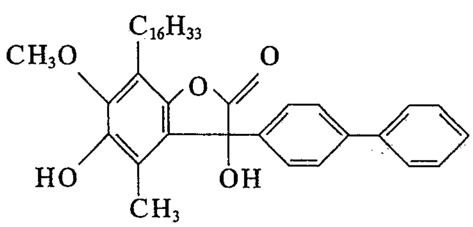

(32)

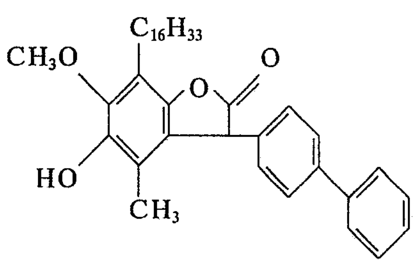

(33)

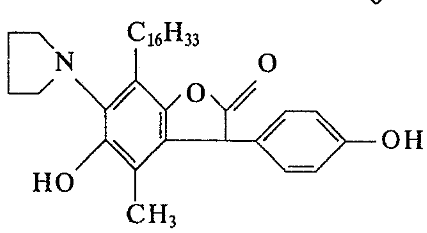

(34)

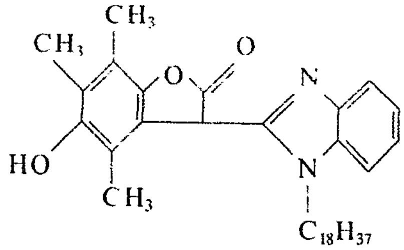

(35)

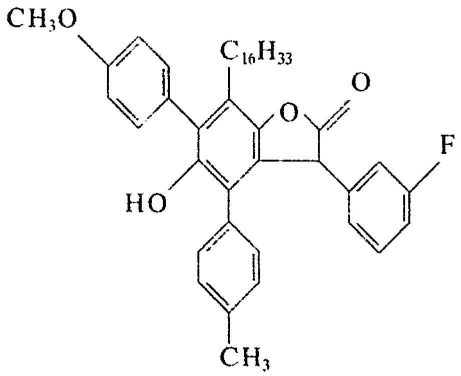

(36)

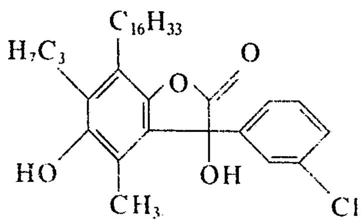

(37)

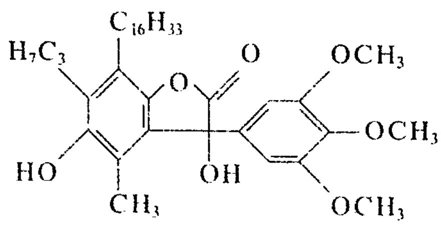

(38)

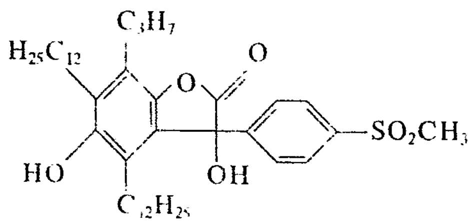

(39)

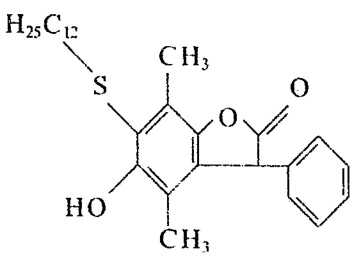

(40)

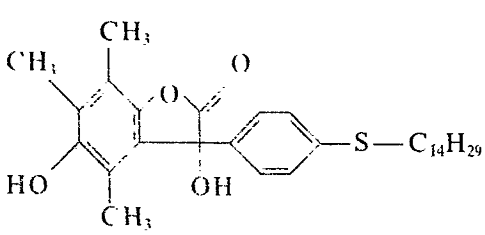

(41)

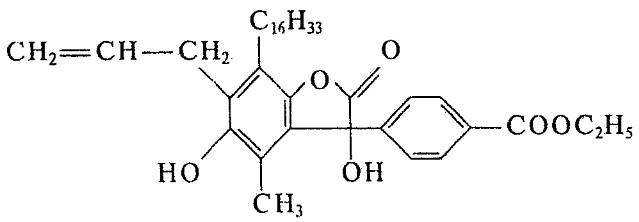
(42)

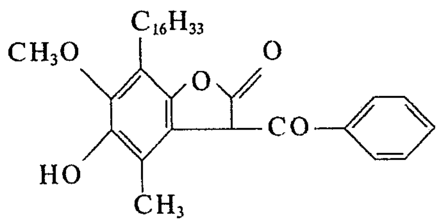
(43)

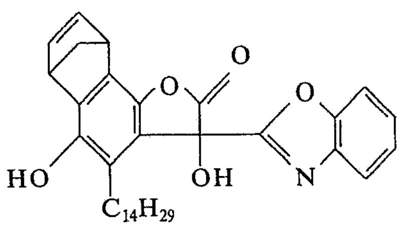
(44)

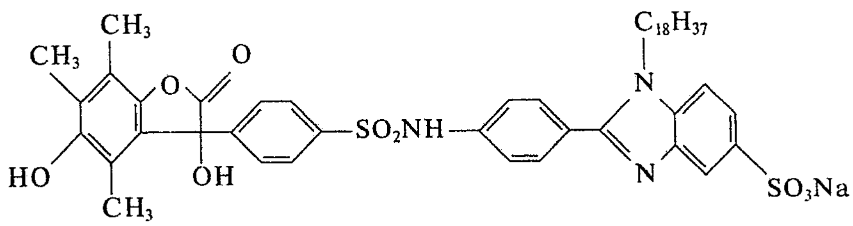
(45)

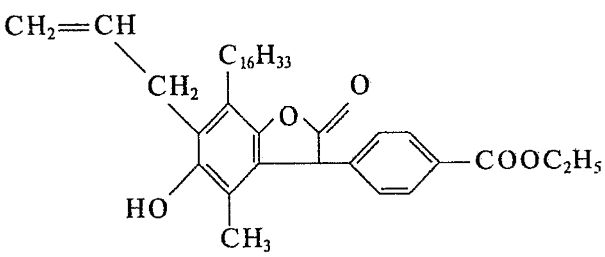
(46)

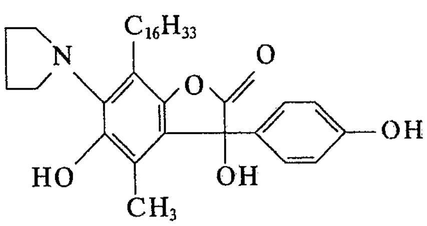
(47)

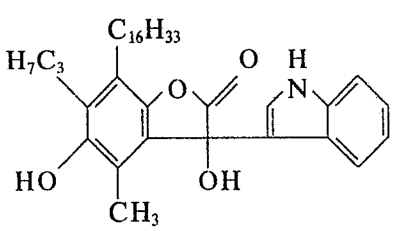
(48)

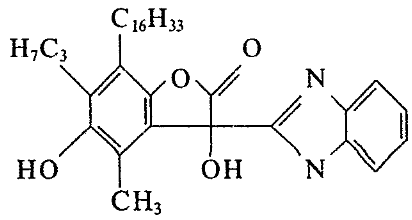

(49)

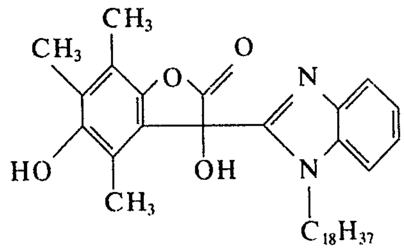

(50)

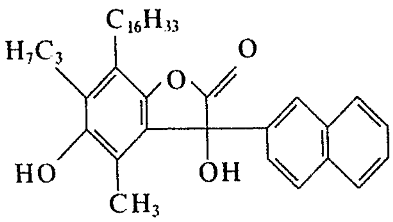

(51)

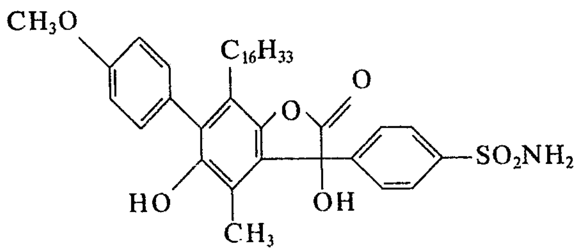

(52)

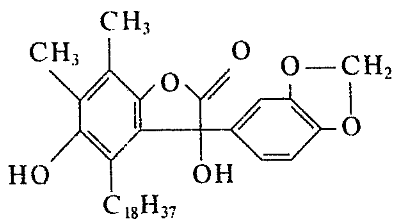

(53)

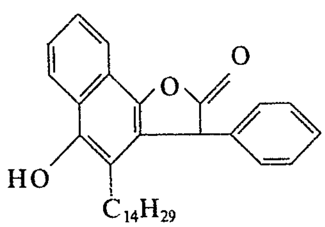

(54)

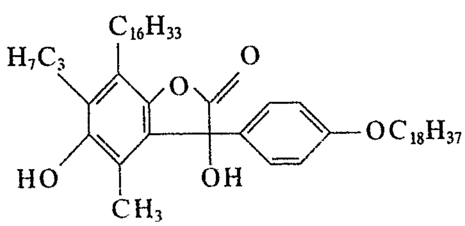

(55)

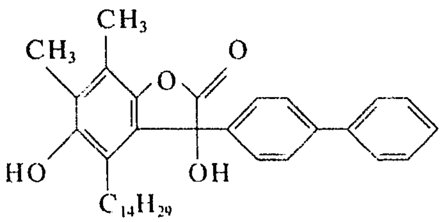

(56)

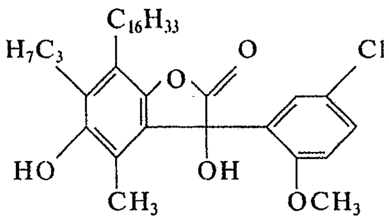

(57)

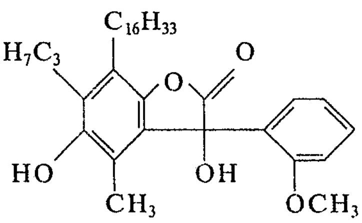

(58)

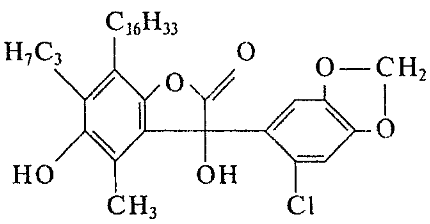

(59)

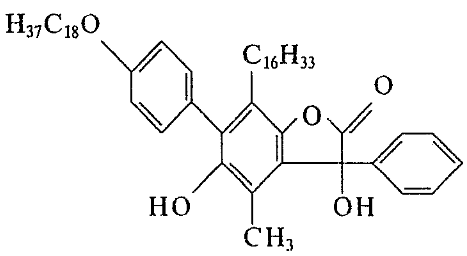

(60)

Die erfindungsgemäß verwendeten ED-Vorläuferverbindungen der Formel I sind als $\alpha$-substituierte $\alpha$-Hydroxylactone von Hydrochinonen mit einem geeigneten $\alpha$-OH-$\alpha$-$R^1$-substituierten Essigsäurerest aufzufassen. Ihre Herstellung gelingt durch Anlagerung von Sauerstoff an die entsprechenden Lactone der allgemeinen Formel II

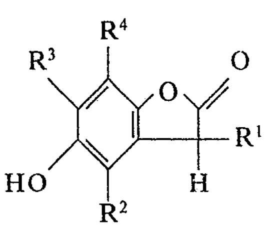

(II)

in der $R^1$, $R^2$, $R^3$ und $R^4$ definiert sind wie bei Formel I angegeben, entsprechend der Herstellung von Triphenylcarbinol aus Triphenylmethan (Lit.: RUSSEL et al, J. Amer. Chem. Soc. 84, 2652 (1962)).

Die Lactone der Formel II sind erhältlich durch Michael-Addition von $\alpha$-$R^1$-substituierten Acetonitrilen an die entsprechenden durch $R^2$, $R^3$ und $R^4$ substituierten p-Benzochinone.

Die verwendeten Acetonitrile (z. B. Benzylcyanide) sind größtenteils im Handel erhältlich oder lassen sich nach üblichen Methoden der organischen Synthese herstellen, [Lit.: 1) HOUBEN-WEYL, Methoden der Organischen Chemie, Band VIII, S. 294, Georg-Thieme-Verlag Stuttgart, 1952; 2) SHIGELIKO SUGASAWA, Hajime Shigehara, Chem. Ber. 74, 459 (1941)].

Die benötigten p-Benzochinone mit $R^2$, $R^3$ und $R^4$ wurden ebenfalls nach literaturbekannten Verfahren hergestellt.

(Lit.: 1) HOUBEN-WEYL, Methoden der Organischen Chemie, Bd. 7/3 a »Chinone I«; 2) SCHILL, ZÜRCHER, LOGEMANN, Chem. Ber. 108, 1570 (1975); 3) KUSER, INDERBITZIN, BRAUCHLI, EUGSTER, Helv. Chim. Acta 54, 980 (1971)).

Nachfolgend wird die Herstellung einiger ausgewählter Verbindungen beschrieben:

Verbindung 1

6,6 g (0,29 Mol) Natrium wurden in 300 ml abs. Ethanol gelöst und unter Rühren mit der Lösung von 59 g (0,37 Mol) Malonsäurediethylester in 60 ml abs. Ethanol versetzt. Nach 10 min Rühren wird im Laufe von 60 min eine Lösung von 115 g (0,3 Mol) 2-Methyl-5-hexadecyl-6-propyl-1,4-benzochinon in 1300 ml abs. Ethanol zugetropft. Es wurde noch 2 Stunden bei Raumtemperatur weitergerührt und der Ansatz über Nacht stehen gelassen.

Die Reaktionsmischung wurde nun auf 5 kg Eis, dem 125 ml konz. Salzsäure zugeführt waren, ausgegossen, der ausfallende Niederschlag mit Essigester aufgenommen. Der Essigextrakt wurde mehrmals mit Wasser gut gewaschen, mit $Na_2SO_4$ sicc. getrocknet und eingeengt. Der verbleibende Rückstand wurde mit Benzin verrührt, abgesaugt.

Man erhielt 111 g = 75% Ausbeute; F: 87–88,5°C.

Verbindung 3

Vorstufe

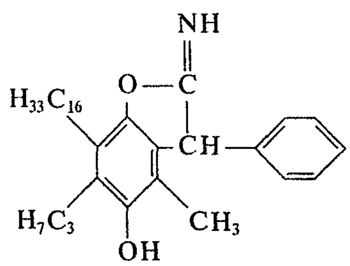

9,5 g (0,412 Mol) Natrium wurden in 400 ml wasserfreiem Ethanol gelöst und mit der Lösung von 62 g (0,53 Mol) Benzylcyanid in 60 ml wasserfreiem Ethanol unter Rühren versetzt. Nach ca. 10minütigem Rühren und Abkühlen auf Raumtemperatur wurde im Laufe von 60 Minuten und unter intensivem Rühren die Lösung von 160 g (0,412 Mol) 2-Methyl-5-hexadecyl-6-propyl-1,4-benzochinon in 1600 ml wasserfreiem Ethanol zugetropft. Nach 3stündigem Rühren bei Raumtemperatur wurde der Ansatz über Nacht stehen gelassen, dann auf ein Gemisch aus 5 l Eiswasser und 100 ml konzentrierter Salzsäure ausgegossen und mit Essigester ausgeschüttelt.

Nach mehrmaligem Waschen mit Wasser und Trocknen mit $Na_2SO_4$ sicc. wurde der Essigesterextrakt am Rotationsverdampfer eingeengt.

Man erhielt ca. 250 g der Vorstufe, die als Rohprodukt weiter umgesetzt wurde.

Verbindung 3

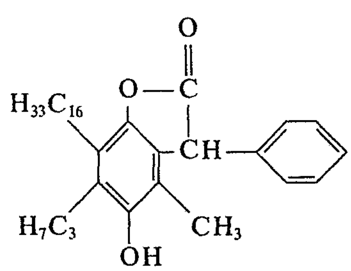

253 g Rohprodukt der Vorstufe wurden in 1,5 l Aceton heiß gelöst und mit 120 ml konzentrierter Salzsäure innerhalb 30 Minuten unter Rühren versetzt. Nach 10minütigem Kochen am Rückfluß wurde die Lösung in Wasser eingerührt und der ausfallende Niederschlag mit Essigester ausgeschüttelt. Die Extrakte wurden mit Wasser gewaschen und über $Na_2SO_4$ sicc. getrocknet und am Rotationsverdampfer zur Trockne eingeengt.

Der Rückstand wurde mit ca. 300 ml Benzin aufgenommen. Der ausfallende Niederschlag wurde abgesaugt und mit Benzin gewaschen. Nach Umkristallisieren aus Methanol/Essigester (40 : 3) erhielt man farblose Kristalle von Fp. 113–115°C.

Ausbeute: 127 g.

Verbindung 4

78 g (0,154 Mol) Verbindung 3 wurden in 1,25 l eines Gemisches aus wasserfreiem DMSO (Dimethylsulfoxid) und t-Butanol (4 : 1) in der Wärme gelöst und abgekühlt. 1,57 g Kalium-t.-butylat wurden hinzugefügt und innerhalb von 60 min wurden ca. 20 l $O_2$-Gas durch das Gemisch geleitet. Die Reaktionslösung wurde dann in ein Gemisch aus 4 l Wasser, 10 ml Eisessig und 1,5 l Essigester eingerührt, die Essigesterphase abgetrennt, mehrmals mit Wasser gewaschen und nach Trocknen über $Na_2SO_4$ sicc. eingedampft.

Der Rückstand wurde in Benzin aufgenommen und der dabei ausfallende Niederschlag abgesaugt. Nach dem Umkristallisieren aus Benzin erhielt an 52,5 g der Verbindung 4 vom Fp. 99—100°C.

Verbindung 10

Stufe 1
4-Octadecyloxybenzylcyanid

Eine Lösung von 55 g 4-Octadecyloxybenzylchlorid in 100 ml Benzol wird bei 50°C mit einer Lösung von 21,5 g Natriumcyanid und 1 g Tetrabutylammoniumbromid in 40 ml Wasser versetzt und 4 Stunden gerührt. Die Benzolschicht wird zweimal mit Wasser gewaschen, getrocknet und abgedampft. Der Rückstand wird aus Benzol (50—75°C) umkristallisiert.
Ausbeute: 47,5 g  Fp. 79—80°C

Das als Ausgangsmaterial benötigte 4-Octadecyloxybenzylchlorid kann man auf folgendem Wege herstellen:

4-Oxybenzaldehyd wird mit Octadecylbromid am Sauerstoff alkyliert, der entstandene 4-Octadecyloxybenzaldehyd (F 48—50°C) wird mit Natriumborhydrid zum 4-Octadecyloxybenzylalkohol (F 83—85°C) reduziert und dieser mit Chlorwasserstoff in benzolischer Lösung in 4-n-Octadecyloxybenzylchlorid (F 52—55°C) übergeführt.

Stufe 2
5-Hydroxy-2-imino-4,6,7-trimethyl-3-(4'-octadecyloxyphenyl)-2,3-dihydro-benzo[b]furan

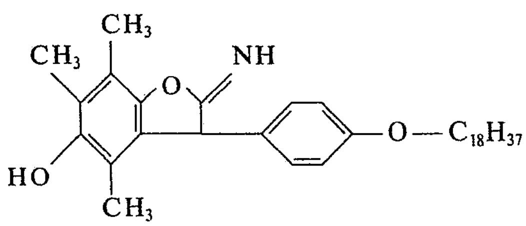

In einem 3-Halskolben mit Rührer, Tropftrichter und Rückflußkühler werden 2,3 g (0,1 Mol) Natrium in 300 ml Ethanol abs. gelöst. Nach Zugabe von 42,3 g (0,11 Mol) ›Benzylcyanid‹ aus Stufe 1 wird unter Rühren bis ca. 75°C erwärmt und eine Lösung von 15 g (0,1 Mol) Trimethyl-p-benzochinon in 130 ml Ethanol abs. in 10—15 Minuten zugetropft, anschließend noch unter langsamer Abkühlung 2 Stunden weitergerührt, nach Stehen über Nacht in ein Gemisch von 1 nHCl/Ethylacetat eingerührt, die Ethylacetatschicht abgetrennt, mit Wasser zweimal ausgeschüttelt und die Ethylacetatlösung eingedampft. Der Rückstand wurde ohne Reinigung weiterverarbeitet.

Verbindung 10
5-Hydroxy-2-oxo-4,6,7-trimethyl-3-(4'-octadecyloxyphenyl)-2,3-dihydro-benzo[b]furan

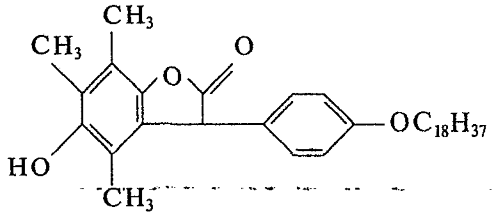

Der Rückstand aus Stufe 2 wird in 650 ml Aceton unter Erwärmen gelöst und nach Zugabe von 32 ml Salzsäure conc. 30 Minuten unter Rückfluß gekocht. Nach Ausgießen auf Wasser wird die organische Phase in Ethylacetat aufgenommen, zweimal mit Wasser gewaschen und abgetropft. Der Rückstand wird mit Petrolether behandelt und das feste Produkt abgesaugt.
Ausbeute: 45 g  Fp. 114—117°C

Verbindung 19

26 g der Verbindung 10 werden in 500 ml eines Gemisches von 4 Teilen Dimethylsulfoxid und 1 Teil tert.-Butanol gelöst und nach Zugabe von 0,54 g Kalium-tert.-butylat bei 20°C ein Sauerstoffstrom (200 ml in 30 s) durchgeleitet. Durch leichtes Kühlen wird die Temperatur auf 20°C gehalten. Nach 60 min ist die Sauerstoffanlagerung vollständig und die Lösung wird auf 0,1 n Essigsäure (1 l) ausgegossen. Nach Aufnahme in Ethylacetat und mehrmaligem Waschen mit Wasser wird die Ethylacetatlösung eingedampft. Der Rückstand wird aus n-Butylchlorid umkristallisiert.
Ausbeute: 14 g Fp. 132–135°C

Für die photographische Brauchbarkeit der in der erfindungsgemäßen Weise zu verwendenden Benzolactone, insbesondere bei Farbabspaltverfahren, ist die Aufspaltung des Lactonringes zur Überführung der ED-Vorläuferverbindung in die ED-Verbindung unter den bei der Verarbeitung gewünschten Bedingungen besonders wichtig. Dabei soll die Aufspaltung bei pH-Werten zwischen 9 und 13 erfolgen – eine Aufspaltung bei niedrigeren pH-Werten würde zu einer verfrühten Bildung der ED-Verbindung und damit zu Farbschleiern führen; erfolgt die Aufspaltung erst bei pH-Werten über 13, bildet sich die ED-Verbindung zu langsam und die Bildungszeiten für das Farbstoffbild werden zu lang. Da die ED-Vorläuferverbindung und die damit in Kombination verwendete nicht diffundierende reduzierbare farbgebende Verbindung in einem der bekannten Ölbildner (oilformer) gelöst und diese Lösung oder Lösungen in die wäßrige Phase der Gießlösung für die photographische Schicht eindispergiert werden, so daß die Verbindungen feinverteilt in der organischen Phase enthalten sind, bezieht sich die Angabe des geeigneten pH-Wertes zwischen 9 und 13 auf den in der wäßrigen Phase.

In der erfindungsgemäßen Weise brauchbare Benzolacton-Verbindungen müssen außer der oben angegebenen Forderung noch eine zweite Bedingung erfüllen. Die sich aus der ED-Vorläuferverbindung durch Aufspaltung des Lactonringes bildende ED-Verbindung muß ein hinreichend niedriges Redoxpotential besitzen. Das Redoxpotential der ED-Verbindung muß, gemessen gegen Normal-Kalomelelektrode kleiner sein als +0,255 V vorzugsweise kleiner als +0,191 V, bei pH = 0. Für die Messung des Redoxpotentials wird die übliche Meßmethode nach IUPAC 1953, beschrieben in »Handbook for Techniques of Electrochemistry«, Vol. 1, Seiten 5 ff., verwendet.

Die durch die elektronenanziehenden Substituenten im Lactonring gesteuerte Hydrolysierbarkeit dieses Ringes wird wie folgt gemessen:

Zunächst wird folgende organische Lösung hergestellt:

40 mg der ED-Vorläuferverbindung
10 mg Phenyl-pyrazolindon-3
10 ml n-Butanol

Diese butanolische Lösung wird geteilt und je zur Hälfte in die wäßrigen Lösungen der folgenden Zusammensetzung eindispergiert:

Lösung A (pH ca. 9,2)

50 mg Kaliumferricyanid
310 mg Borsäure
373 mg Kaliumchlorid
85 mg Natriumhydroxid
15 ml Wasser

Lösung B (pH ca. 13,2)

50 mg Kaliumferricyanid
373 mg Kaliumchlorid
188 mg Natriumhydroxid
15 ml Wasser

Die Aufspaltung des Lactonringes und gleichzeitige Oxidation des Hydrochinon- zum entsprechenden Chinon-Derivat kann sowohl kontinuierlich auf spektroskopischem Wege als auch durch laufende Probenentnahme und DC-analytische Untersuchung verfolgt und gemessen werden. Die Messung wird bei Zimmertemperatur und unter intensiver Rührung durchgeführt.

Brauchbar sind solche ED-Vorläuferverbindungen der erfindungsgemäßen Konstitution, bei denen die Auspaltung und Oxidation in Lösung A mit einer Halbwertszeit >10 min und in Lösung B mit einer Halbwertszeit <10 min erfolgt.

Nachfolgend die mit den beispielhaften Verbindungen erhaltenen Werte:

| Verbindung Nr. | A | B |
|---|---|---|
| 1 | >10 min | <10 s |
| 3 | >10 min | <10 s |
| 4 | >10 min | <15 s |
| 10 | >10 min | <10 s |
| 19 | >10 min | <10 s |

Die erfindungsgemäßen ED-Vorläuferverbindungen sind den bekannten ED-Verbindungen, z. B. Ascorbylpalmitat, sowie auch den bekannten ED-Vorläuferverbindungen insofern überlegen, als sich mit ihnen innerhalb einer gegebenen Entwicklungszeit höhere Maximalfarbdichten bei gleichzeitig geringeren Minimalfarbdichten (Schleier) erzeugen lassen. Es mag dabei dahingestellt bleiben, ob die erhaltenen höheren Maximalfarbdichten auf eine leichtere Öffnung des Lactonringes und damit auf eine raschere Verfügbarkeit der eigentlichen ED-Verbindungen oder auf deren höheres Reduktionsvermögen oder aber auf beide Einflüsse zurückzuführen ist.

Als nicht diffundierende reduzierbare farbgebende Verbindungen, die mit den erfindungsgemäßen ED-Vorläuferverbindungen in Kombination verwendet werden können, eignen sich beispielsweise die sogenannten BEND-Verbindungen der DE-A- 2 809 716 (BEND = ballasted electron-accepting nucleophilic displacement). Diese Verbindungen erfordern zur Farbstoff-Freisetzung eine durch die Reduktion ermöglichte intramolekulare nukleophile Verdrängungsreaktion. Ein anderer Typ von ebenfalls geeigneten nicht diffundierenden reduzierbaren farbgebenden Verbindungen ist Gegenstand der offengelegten europäischen Patentanmeldung 0 004 399.

Das erfindungsgemäße farbphotographische Aufzeichnungsmaterial enthält nun bei monochromatischen Verfahren mindestens eine, bei Verfahren zur Herstellung mehrfarbiger Bilder in der Regel mindestens drei bilderzeugende Schichteinheiten, von denen jede mindestens eine lichtempfindliche Silberhalogenidemulsionsschicht und eine dieser zugeordnete Kombination aus einer nicht diffundierenden reduzierbaren farbgebenden Verbindung und einer Elektronendonorverbindung enthält, wobei in mindestens einer Schichteinheit eine erfindungsgemäße ED-Vorläuferverbindung verwendet wird. In der Regel ist eine der Schichteinheiten überwiegend für blaues Licht empfindlich, eine weitere für grünes Licht und eine dritte für rotes Licht, wobei die zugeordneten farbgebenden Verbindungen jeweils komplementärfarbige Bildfarbstoffe liefern.

Unter »Zuordnung« und »zugeordnet« wird verstanden, daß die gegenseitige Anordnung von Silberhalogenidemulsionen, ED-Verbindung bzw. ED-Vorläuferverbindung und farbgebender Verbindung von solcher Art ist, daß eine Wechselwirkung zwischen ihnen möglich ist, die eine bildmäßige Übereinstimmung zwischen dem gebildeten Silberbild und dem Verbrauch an ED-Verbindung einerseits sowie zwischen der nicht verbrauchten ED-Verbindung und der farbgebenden Verbindung andererseits zuläßt, so daß in Übereinstimmung mit dem nicht entwickelten Silberhalogenid eine bildweise Verteilung von diffundierendem Farbstoff erzeugt wird. Hierzu müssen lichtempfindliches Silberhalogenid und Kombination aus farbgebender Verbindung und ED-Verbindung nicht notwendigerweise in derselben Schicht vorliegen; sie können auch in zueinander benachbarten Schichten untergebracht sein, die jeweils der gleichen Schichteinheit angehören.

Um eine hinreichende Wechselwirkung zwischen der farbgebenden Verbindung und der zugeordneten ED-Vorläuferverbindung zu gewährleisten, empfiehlt es sich, diese beiden Verbindungen einer Kombination in der gleichen Schicht unterzubringen, die jedoch nicht mit der zugeordneten Silberhalogenidemulsionsschicht identisch sein muß. Die Tatsache, daß die erfindungsgemäßen ED-Vorläuferverbindungen unter neutralen Bedingungen hydrolysestabil und somit auch oxidationsunempfindlich sind, macht diese Verbindungen besonders geeignet für die Verwendung in einem gemeinsamen Emulgat (zusammen mit der farbgebenden Verbindung), während übliche ED-Verbindungen unter vergleichbaren Bedingungen eine zu hohe Oxidationsempfindlichkeit aufweisen, so daß sie nicht mit den farbgebenden Verbindungen in dem gleichen Emulgat verwendet werden können.

Für die Einarbeitung der erfindungsgemäßen ED-Vorläuferverbindungen sind alle Methoden geeignet, nach denen üblicherweise hydrophobe Verbindungen in photographischen Schichten eingearbeitet werden, d. h. es kommen die üblichen Emulgiertechniken in Frage, z. B. Methoden, nach denen photographische Hilfsstoffe den Gießlösungen in Form von Emulgaten unter Verwendung sogenannter Ölbildner zugesetzt werden. Zu vermeiden sind hierbei zweckmäßigerweise alle Methoden, bei denen die Verwendung von Alkali unvermeidlich ist.

Die nicht diffundierende reduzierbare farbgebende Verbindung wird in einer Schicht in der Regel in einer solchen Menge verwendet, die ausreicht, um ein Farbbild mit möglichst hoher maximaler Farbdichte zu erzeugen, beispielsweise in einer Menge von $1-20 \cdot 10^{-4}$ Mol/$m^2$. Die erfindungsgemäße ED-Vorläuferverbindung ist in der Menge an diejenige der farbgebenden Verbindung angepaßt. Sie soll groß genug sein, um eine möglichst hohe maximale Farbdichte zu erzielen, d. h. um eine möglichst vollständige Reduktion der farbgebenden Verbindung bewirken zu können. Andererseits soll sie auch nicht wesentlich höher sein als hierzu erforderlich, so daß das aus ihr resultierende Reduktionsmittel an den belichteten Stellen durch die Entwicklung des belichteten Silberhalogenids möglichst vollständig verbraucht werden kann. Die im Einzelfall günstigsten Mengenverhältnisse zwischen Silberhalogenid, ED-Vorläuferverbindung und farbgebender Verbindung werden zweckmäßigerweise anhand von Routineversuchen ermittelt. Brauchbare Ergebnisse können beispielsweise erhalten werden, wenn die ED-Vorläuferverbindung jeweils in der 1- bis 5-fachen molaren Menge, bezogen auf die farbgebende Verbindung, vorliegt. Das geeignete Mengenverhältnis zwischen Silberhalogenid und zugeordneter farbgebender Verbindung liegt etwa im Bereich von 2—20 Mol Silberhalogenid pro Mol farbgebender Verbindung.

Zwischen verschiedenen Schichteinheiten sind zweckmäßigerweise Trennschichten vorhanden, die Verbindungen enthalten können, die mit diffundierenden Entwicklungsprodukten zu reagieren und deren Diffusion aus einer Schichteinheit in eine andere zu unterbinden vermögen. Dies trägt dazu bei, daß die Zuordnung jeweils auf eine Schichteinheit begrenzt bleibt. Derartige Verbindungen sind bekannt, hierfür eignen sich beispielsweise nicht diffundierende Hydrochinonderivate sowie beispielsweise die in der Publikation »Research Disclosure No. 17 842« (Februar 1979) beschrieben »scavenger compounds«. Nicht zuletzt können auch die erfindungsgemäßen ED-Vorläuferverbindungen diese Funktion übernehmen, wenn sie in eine Trennschicht zwischen verschiedenen Schichteinheiten eingelagert werden. Die Wechselwirkung zwischen dem belichteten Silberhalogenid und der aus der erfindungsgemäßen nicht diffundierenden ED-Vorläuferverbindung unter den Bedingungen der alkalischen Entwicklung entstehenden nicht diffundierenden ED-Verbindung wird im allgemeinen durch die oxidierte Form des zur Anwendung gelangenden Silberhalogenidentwicklungsmittels bewerkstelligt. Letzteres wird bei der Entwicklung bildmäßig oxidiert und das Oxidationsprodukt ist seinerseits in der Lage, die ED-Verbindung zu oxidieren und damit der Reaktion mit der farbgebenden Verbindung zu entziehen. Die Wechselwirkung zwischen der nicht oxidierten ED-Verbindung und der farbgebenden Verbindung erfolgt direkt.

Typische geeignete Elektronenübertragungsmittel sind beispielsweise Hydrochinonverbindungen, z. B. Hydrochinon, 2,5-Dichlorhydrochinon und 2-Chlorhydrochinon; Aminophenolverbindungen wie beispielsweise 2-Aminophenol, N-Methylaminophenol, 3-Methyl-4-aminophenol sowie 3,5-Dibromaminophenol; Brenzkatechinverbindungen wie beispielsweise Brenzkatechin, 4-Cyclohexylbrenzkatechin, 3-Methoxybrenzkatechin und 4-(N-Octadecylamino)-brenzkatechin; Phenylendiaminverbindungen wie beispielsweise N,N-Diethyl-p-phenylendiamin, 3-Methyl-N,N-diethyl-p-phenylendiamin, 3-Methoxy-N-ethyl-N-hydroxyethyl-p-phenylendiamin sowie N,N,N',N'-Tetramethyl-p-phenylendiamin.

Gemäß einer besonders vorteilhaften Ausgestaltung der Erfindung wird als Elektronenübertragungsmittel eine 3-Pyrazolidonverbindung verwendet, nämlich beispielsweise:

1-Phenyl-3-pyrazolinon, 1-Phenyl-4,4-dimethyl-3-pyrazolidon,
4-Hydroxymethyl-4-methyl-1-phenyl-3-pyrazolidon, 1-p-Tolyl-3-pyrazolidon,
1-Phenyl-4-methyl-3-pyrazolidon, 1-Phenyl-5-methyl-3-pyrazolidon,
1-Phenyl-4,4-bis-(hydroxymethyl)-3-pyrazolidon, 1,4-Dimethyl-3-pyrazolidon,
4-Methyl-3-pyrazolidon, 4,4-Dimethyl-3-pyrazolidon,
1-(3-Chlorphenyl)-4-methyl-3-pyrazolidon, 1-(4-Chlorphenyl)-4-methyl-3-pyrazolidon,
1-(3-Chlorphenyl)-3-pyrazolidon, 1-(4-Chlorphenyl)-3-pyrazolidon,
1-(4-Tolyl)-4-methyl-3-pyrazolidon, 1-(2-Tolyl)-4-methyl-3-pyrazolidon,
1-(4-Tolyl)-4-hydroxymethyl-4-methyl-3-pyrazolidon, 1-(3-Tolyl)-3-pyrazolidon,
1-(3-Tolyl)-4,4-dimethyl-3-pyrazolidon, 1-(2-Trifluorethyl)-4,4-dimethyl-3-pyrazolidon und
5-Methyl-3-pyrazolidon.

Auch läßt sich eine Kombination von verschiedenen Elektronenübertragungsmitteln verwenden, z. B. eine Kombination, wie sie in US-A- 3 039 869 beschrieben ist.

Die Entwicklerverbindungen können in der Entwicklungsflüssigkeit zur Anwendung gebracht werden oder können, mindestens teilweise, in irgendeiner Schicht des photographischen Aufzeichnungsmaterials untergebracht werden, z. B. in einer oder mehreren Silberhalogenidemulsionsschichten, Schichten, in denen die farbgebenden Verbindungen untergebracht werden, Zwischenschichten oder Bildempfangsschichten.

Die im Einzelfalle optimale Elektronenübertragungsverbindung hängt natürlich von der im Einzelfalle verwendeten ED-Verbindung ab sowie der nicht diffundierenden reduzierbaren farbgebenden Verbindung sowie den Entwicklungsbedingungen des verwendeten Aufzeichnungsmaterials.

Zur Durchführung des Farbstoffdiffusionsübertragungsverfahrens wird in der Regel ein lichtempfindliches Element verwendet, das eine oder mehrere Silberhalogenidemulsionsschichteinheiten sowie die

diesen zugeordneten nicht diffundierenden farbgebenden Verbindungen und ED-Verbindung bzw. deren Vorläufer enthält, und ein Bildempfangselement, in dem durch die bildmäßige Übertragung diffundierender Farbstoffe das gewünschte Farbbild erzeugt wird. Hierzu ist es erforderlich, daß zwischen dem lichtempfindlichen Element und dem Bildempfangselement mindestens während eines endlichen Zeitraumes innerhalb der Entwicklungszeit ein fester Kontakt besteht, so daß die in dem lichtempfindlichen Element als Folge der Entwicklung erzeugte bildmäßige Verteilung an diffundierenden Farbstoffen auf das Bildempfangselement übertragen werden kann. Der Kontakt kann hergestellt werden, nachdem die Entwicklung in Gang gesetzt worden ist, oder er kann bereits hergestellt worden sein, bevor die Entwicklung beginnt. Letzteres ist beispielsweise der Fall, falls zur Durchführung des Farbstoffdiffusionsübertragungsverfahrens ein sogenanntes integrales Aufzeichnungsmaterial verwendet wird, in dem das lichtempfindliche Element und das Bildempfangselement eine integrale Einheit bilden, im folgenden auch als Monoblattmaterial bezeichnet, die auch nach Beendigung des Entwicklungsvorgangs weiter bestehen bleibt, d. h., eine Abtrennung des lichtempfindlichen Elements vom Bildempfangselement ist auch nach erfolgtem Farbübertrag nicht vorgesehen. Eine solche Ausführungsform ist beispielsweise beschrieben in der DE-A- 2 019 430.

Das Bildempfangselement kann demnach Bestandteil des farbphotographischen Aufzeichnungsmaterials sein, z. B. in Gestalt einer Bildempfangsschicht, die unterhalb der lichtempfindlichen Silberhalogenidemulsionsschichteinheiten auf einem transparenten Träger angeordnet ist, wobei sich zweckmäßigerweise zwischen der Bildempfangsschicht und den lichtempfindlichen Schichten eine lichtundurchlässige, vorzugsweise lichtreflektierende Bindemittelschicht befindet. Die Bildempfangsschicht, die bei einer anderen Ausgestaltung des Farbübertragungsverfahrens aber auch auf einen separaten Träger angeordnet sein kann (Bildempfangsblatt), enthält in der Regel in bekannter Weise ein basisches Beizmittel für diffundierende anionische (saure) Farbstoffe.

Als Beizmittel für saure Farbstoffe dienen vorzugsweise langkettige quaternäre Ammonium- oder Phosphoniumverbindungen oder tertiäre Sulfoniumverbindungen, z. B. solche, wie sie beschrieben sind in US-A- 3 271 147 und US-A- 3 271 148. Ferner können auch bestimmte Metallsalze und deren Hydroxide, die mit den sauren Farbstoffen schwerlösliche Verbindungen bilden, verwendet werden. Die Farbstoffbeizmittel sind in der Empfangsschicht in einem der üblichen hydrophilen Bindemittel dispergiert, z. B. in Gelatine, Polyvinylpyrrolidon, ganz oder partiell hydrolysierten Celluloseestern. Selbstverständlich können auch manche Bindemittel als Beizmittel fungieren, z. B. Mischpolymerisate oder Polymerisatgemische von Vinylalkohol und N-Vinylpyrrolidon, wie beispielsweise beschrieben in der DE-A- 1 130 284, ferner solche, die Polymerisate von stickstoffhaltigen quaternären Basen darstellen, z. B. Polymerisate von N-Methyl-2-vinylpyridin, wie beispielsweise beschrieben in US-A- 2 484 430. Weitere brauchbare beizende Bindemittel sind beispielsweise Guanylhydrazonderivate von Alkylvinylketonpolymerisaten, wie beschrieben in US-A- 2 882 156, oder Guanylhydrazonderivate von Acylstyrolpolymerisaten, wie beispielsweise beschrieben in DE-A- 2 009 498.

Im allgemeinen wird man jedoch auch den zuletzt genannten beizenden Bindemitteln andere Bindemittel, z. B. Gelatine, zusetzen. Weitere polymere Beizmittel sind beschrieben etwa in US-A- 3 709 690, DE-A- 2 315 304, DE-A- 2 445 782, DE-A- 2 551 786 und DE-A- 2 631 521.

Das erfindungsgemäße farbphotographische Aufzeichnungsmaterial kann darüber hinaus noch saure Schichten und sogenannte Brems- oder Verzögerungsschichten enthalten, die zusammen ein sogenanntes integrales Neutralisationssystem bilden. Ein solches integrale Neutralisationssystem kann in bekannter Weise zwischen dem Schichtträger und der darauf angeordneten Bildempfangsschicht angeordnet sein oder an einer anderen Stelle im Schichtverband, z. B. oberhalb der lichtempfindlichen Schichten, d. h. jenseits dieser lichtempfindlichen Schichten, von der Bildempfangsschicht aus betrachtet. Das Neutralisationssystem ist normalerweise so orientiert, daß sich die Brems- oder Verzögerungsschicht zwischen der Säureschicht und der Stelle befindet, an der die alkalische Entwicklungsflüssigkeit oder -paste zur Einwirkung gebracht wird. Solche Säureschichten, Bremsschichten usw. aus beiden bestehenden Neutralisationssystemen sind beispielsweise bekannt aus
US-A- 2 584 030, US-A- 2 983 606, US-A- 3 362 819, US-A- 3 362 821,
DE-A- 2 455 762, DE-A- 2 601 653, DE-A- 2 716 505, DE-A- 2 816 878.
Ein solches Neutralisationssystem kann in bekannter Weise auch zwei oder mehrer Bremsschichten enthalten.

Das erfindungsgemäße Aufzeichnungsmaterial kann in einer besonderen Ausgestaltung des weiteren eine oder mehrere für wäßrige Flüssigkeiten durchlässige pigmenthaltige opake Schichten enthalten. Diese können zwei Funktionen erfüllen:

Einerseits kann hierdurch der unerwünschte Zutritt von Licht zu lichtempfindlichen Schichten unterbunden werden und andererseits kann eine solche Pigmentschicht — insbesondere, wenn ein helles oder weißes Pigment, z. B. $TiO_2$ verwendet wird — für das erzeugte Farbbild einen ästhetisch angenehmen Hintergrund bilden. Integrale farbphotographische Aufzeichnungsmaterialien mit einer solchen Pigmentschicht sind bekannt, z. B. aus US-A- 2 543 181 und DE-A- 1 924 430. Anstelle einer vorgebildeten opaken Schicht können auch Mittel vorgesehen sein, um eine solche Schicht erst im Verlauf des Entwicklungsverfahrens zu erzeugen. Entsprechend den beiden erwähnten Funktionen können derartige Pigmentschichten aus zwei oder mehreren Teilschichten aufgebaut sein, von denen eine beispielsweise ein weißes Pigment und die andere beispielsweise ein dunkles, lichtabsorbierendes Pigment, z. B. Ruß enthält.

Bei einer besonders bevorzugten Ausgestaltung der Erfindung ist das photographische Material ein integrales farbphotographisches Ausfzeichnungsmaterial für die Durchführung des Farbdiffusionsübertragungsverfahrens und weist beispielsweise folgende Schichtelemente auf:

1) einen transparenten Schichtträger
2) eine Bildempfangsschicht
3) eine lichtundurchlässige Schicht
4) ein lichtempfindliches Element mit mindestens einer lichtempfindlichen Silberhalogenidemulsionsschicht und mindestens einer dieser zugeordneten nicht diffundierenden farbgebenden Verbindung sowie einer ED-Vorläuferverbindung
5) eine Verzögerungsschicht
6) eine saure Polymerschicht
7) einen transparenten Schichtträger

Das Monoblattmaterial kann dabei in der Weise zusammengesetzt werden, daß getrennt voneinander zwei verschiedene Teile hergestellt werden, nämlich der lichtempfindliche Teil (Schichtelemente 1—4) und das Abdeckblatt (Schichtelemente 5—7), die dann schichtseitig aufeinandergelegt und miteinander verbunden werden, gegebenenfalls unter Verwendung von Abstandstriefen, so daß zwischen den beiden Teilen ein Raum für die Aufnahme einer genau bemessenen Menge einer Entwicklungsflüssigkeit gebildet wird. Die Schichtelemente 5 und 6, die zusammen das Neutralisationssystem bilden, können auch, allerdings in vertauschter Reihenfolge, zwischen dem Schichtträger und der Bildempfangsschicht des lichtempfindlichen Teils angeordnet sein.

Es können weiterhin Mittel vorgesehen sein, um eine Entwicklungsflüssigkeit zwischen den lichtempfindlichen Teil und das Abdeckblatt einzuführen, z. B. in Form eines seitlich angeordneten, aufspaltbaren Behälters, der bei Einwirkung mechanischer Kräfte seinen Inhalt zwischen zwei benachbarte Schichten des Monoblattmaterials ergießt.

Die Entwicklerzubereitung kann außer den wäßrigen Alkali noch Entwicklerverbindungen enthalten, die jedoch auf die Art der farbgebenden Verbindungen abzustimmen sind. Weitere mögliche Bestandteile der Entwicklerzubereitung sind Verdickungsmittel zur Steigerung der Viskosität, z. B. Hydroxyethylcellulose, Silberhalogenidlösungsmittel, z. B. Natriumthiosulfat oder eine der in DE-A- 2 126 661 beschriebenen Bis-sulfonylalkan-Verbindungen, Trübungsmittel zur Erzeugung von opaken Schichten, z. B. Pigmente von $TiO_2$, ZnO, Bariumstearat oder Kaolin. Alternativ oder zusätzlich können einige dieser Bestandteile auch in eine oder mehrere Schichten des erfindungsgemäßen farbphotographischen Aufzeichnungsmaterials eingelagert sein.

## Beispiele

Auf einen transparenten Schichtträger aus Cellulosetriacetat wurden in folgender Reihenfolge eine Beizschicht, eine lichtreflektierende Schicht und eine lichtempfindliche Silberhalogenidemulsionsschicht aufgetragen:

### Beizschicht

3,75 g eines Copolymerisats aus einem Teil Styrol und einem Teil Maleinsäureimid von N,N-Dimethyl-N-hexadecyl-N-$\omega$-aminopropyl-ammoniumbromid wurden in 15 ml Ethanol gelöst und diese Lösung wurde in 75 ml 5%iger Gelatinelösung eingerührt und homogenisiert. Nach Zusatz von 2,6 ml 5%iger Saponinlösung und 1 ml 2%iger wäßriger Mucochlorsäurelösung wurde auf eine übliche Gießviskosität (ca. 11 mPa · s) eingestellt und die Lösung wurde bei 40°C im Tauchverfahren auf den Träger aufgetragen (Gießgeschwindigkeit 5 m/min).

### Lichtreflektierende Schicht

Eine Aufschlämmung aus 42 g $TiO_2$ in 20 ml Wasser wurde unter Zusatz von 5 ml 5%iger wäßriger Lösung von Natriumdodecylbenzolsulfonat und 5 ml 5%iger Saponinlösung in 150 ml 8%iger wäßriger Gelatinelösung dispergiert. Nach Zugabe von 1 ml 2%iger Mucochlorsäurelösung wurde die Dispersion auf eine Viskosität von 13 mPa · s bei 40°C eingestellt und im Tauchverfahren auf die getrocknete Beizschicht aufgetragen (Gießgeschwindigkeit 5 m/min).

### Silberhalogenidemulsionsschicht

1 mMol nicht diffundierende farbgebende Verbindung (Farbabspalter) und 1,5 mMol ED-Vorläufer verbindung wurden in 5 ml Essigsäureethylester gelöst und nach Zugabe von 2 ml Palmitinsäurediethylamid in 25 ml 5%iger Gelatinelösung unter Zusatz von 5 ml 5%iger wäßriger Lösung von Natriumdodecylbenzolsulfonat in einem Bühler-Homogenisator bei ca. 1000 U/min einemulgiert. Das Emulgat wurde über ein Faltenfilter filtriert und mit 5%iger Gelatinelösung auf 75 ml aufgefüllt.

Nach Zugabe von 1 ml 2%iger Mucochlorsäurelösung wurde das Emulgat mit 32 g einer gießfertigen Silberbromidiodid-Emulsion versetzt. Diese war hergestellt worden mit 74 g $AgNO_3$/kg Emulsion; sie hatte ein Ag/Gelatine-Verhältnis von 1 : 1,1. Die Silberbromidemulsion enthielt 0,67 Mol-% Silberiodid.

Die Mischungen wurden im Tauchverfahren mit einer Geschwindigkeit von 5 m/min bei ca. 40°C auf die vorbeschriebene Unterlage aufgetragen.

Die verschiedenen Proben wurden nach 24stündigem Trocknen durch ein Graustufenfilter auf der Emulsionsseite belichtet und mit einer ca. 300 µm stark aufgetragenen Entwicklerpaste der nachfolgend beschriebenen Zusammensetzung 2 min bei 18°C entwickelt, 2 min in einer 5%igen Essigsäurelösung gestoppt, danach kurz gewässert und getrocknet.

Entwickler

In 800 ml Wasser wurden 20 g Carbethoxymethylcellulose unter Rühren gelöst. Zu der homogenen Lösung wurden 40 g NaOH fest, 1,5 g Ethylendiamintetraessigsäure-Natriumsalz, 11,5 g Borax, 1 g Natriumhexametaphosphat, 3 g KBr, 1,6 g 1-Phenyl-4-methyl-4-hydroxymethyl-pyrazolidon-3, 0,1 g 1-Phenyl-5-mercapto-tetrazol hinzugefügt. Die Lösung wurde nun mit Wasser auf 1000 ml aufgefüllt und anschließend durch Zugabe von 30 ml Eisessig ein pH-Wert von 13,2—13,3 eingestellt.

Für die folgenden Vergleiche wurde die folgenden farbgebenden Verbindungen herangezogen:

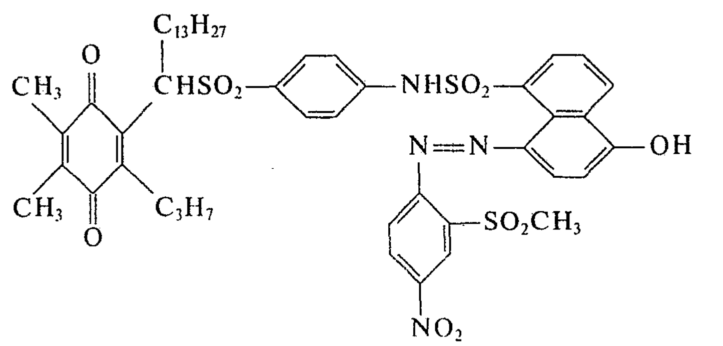

(A)

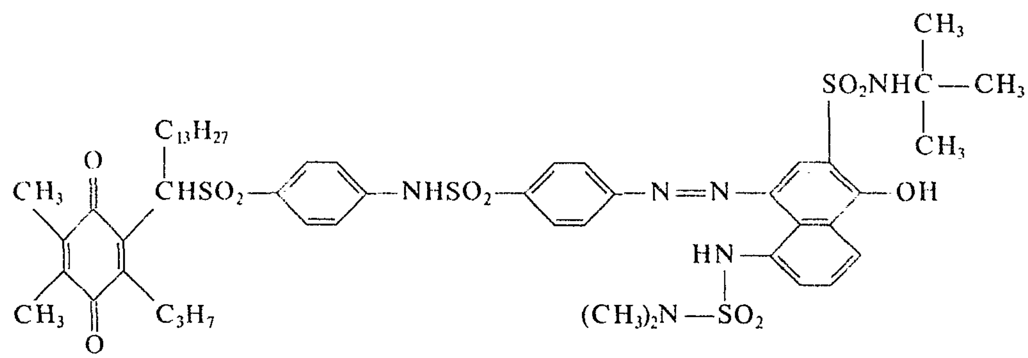

(B)

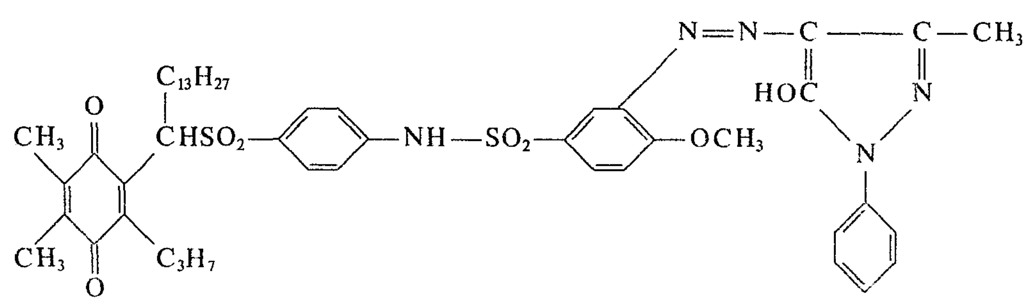

(C)

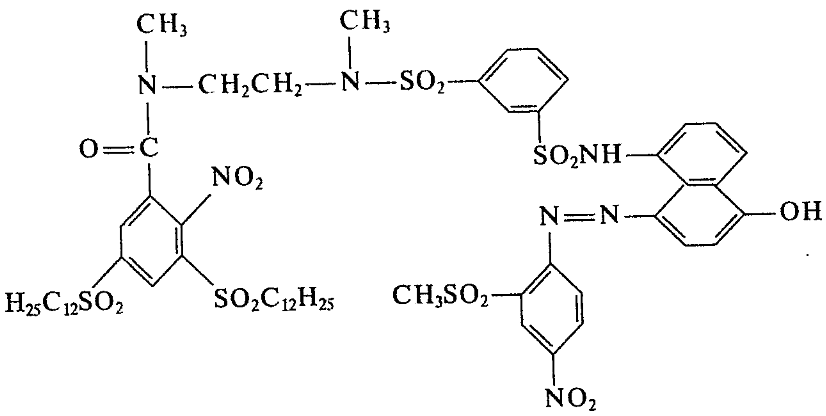

Als ED-Vorläuferverbindung des Standes der Technik (DE-OS 2 806 716) wurde zum Vergleich herangezogen:

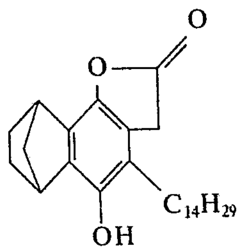

(E)

Nachfolgende Kombinationen wurden auf die erzielbaren $D_{min}/D_{max}$-Werte überprüft:

| Farbstoff | ED-Vorläufer | $D_{min}$ | $D_{max}$ |
|---|---|---|---|
| A | E | 0,04 | 0,60 |
| | 1 | 0,10 | 1,42 |
| | 3 | 0,05 | 1,28 |
| | 4 | 0,04 | 1,92 |
| | 11 | 0,02 | 1,25 |
| | 17 | 0,02 | 2,02 |
| | 10 | 0,02 | 1,08 |
| | 19 | 0,02 | 1,98 |
| B | E | 0,02 | 0,06 |
| | 1 | 0,02 | 1,40 |
| | 3 | 0,02 | 1,05 |
| | 4 | 0,04 | 1,72 |
| | 6 | 0,04 | 1,85 |
| | 20 | 0,02 | 1,12 |
| | 29 | 0,04 | 1,73 |
| | 5 | 0,02 | 1,56 |
| | 12 | 0,05 | 1,95 |
| C | E | 0,0 | 0,06 |
| | 3 | 0,02 | 0,72 |
| | 4 | 0,0 | 0,94 |
| | 10 | 0,02 | 0,82 |
| | 19 | 0,01 | 1,06 |
| D | 3 | 0,06 | 0,95 |
| | 4 | 0,10 | 1,95 |
| | 18 | 0,08 | 0,92 |
| | 53 | 0,10 | 1,64 |

A und D jeweils hinter einem Rotfilter, B hinter Grünfilter und C hinter Blaufilter gemessen.

**Patentansprüche**

1. Farbphotographisches Aufzeichnungsmaterial mit mindestens einer lichtempfindlichen Silberhalogenidemulsionsschicht und einer dieser zugeordneten nicht diffundierenden Elektronendonor-Vorläuferverbindung (ED-Vorläuferverbindung), aus der unter den alkalischen Entwicklungsbedingungen eine nicht diffundierende Elektronendonor-Verbindung (ED-Verbindung) gebildet wird, dadurch gekennzeichnet, daß das Aufzeichnungsmaterial als ED-Vorläuferverbindung ein 5gliedriges $\alpha$-Lacton des Hydrochinons enthält, dessen Lactonring elektronenanziehende Substituenten enthält, die bei pH-Werten zwischen 10 und 13 eine Aufspaltung des Lactonringes unter Bildung einer ED-Verbindung mit einem Redoxpotential von weniger als + 0,255 V (gemessen bei pH 0 gegen eine Normal-Kalomelektrode) bewirken, wobei die Aufspaltung des Lactonringes, gemessen in Halbwertzeiten für den oxidativen Abbau der Lactonverbindung, in einer heterodispersen Mischung aus einer Lösung von

20 mg der ED-Vorläuferverbindung
5 mg 1-Phenyl-pyrazolidon-3 in
5 ml n-Butanol

als feinverteilte organische Phase und einer Lösung von

50 mg Kaliumferricyanid
10 ml Wasser und
5 ml einer Pufferlösung

bei Zimmertemperatur in einer ersten Messung mehr als 10 min und in einer zweiten Messung weniger als 10 min beträgt, wenn die erste Messung unter Verwendung einer Pufferlösung bestehend aus

3,092 g Borsäure ($H_3BO_3$)
3,728 g Kaliumchlorid
0,852 g Natriumhydroxid
ad 50 ml Wasser

und die zweite Messung unter Verwendung einer Pufferlösung bestehend aus

3,728 g Kaliumchlorid
1,884 Natriumhydroxid
ad 50 ml Wasser

durchgeführt wird.

2. Farbphotographisches Aufzeichnungsmaterial mit mindestens einer lichtempfindlichen Silberhalogenidemulsionsschicht und einer dieser zugeordneten nicht diffundierenden Elektronendonor-Vorläuferverbindung (ED-Vorläuferverbindung), aus der unter alkalischen Entwicklungsbedingungen eine nicht diffundierende Elektronendonor-Verbindung (ED-Verbindung) mit einem hohen Reduktionsvermögen gebildet wird, dadurch gekennzeichnet, daß das Aufzeichnungsmaterial als ED-Vorläuferverbindung eine Verbindung der folgenden allgemeinen Formel enthält:

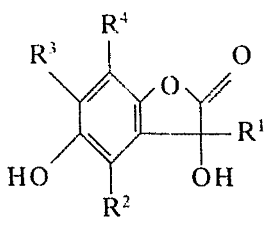

worin bedeuten:

$R^1$ einen carbocyclischen oder heterocyclischen aromatischen Rest;
$R^2$, $R^3$, $R^4$ gleiche oder verschiedene Substituenten, und zwar Wasserstoff, Alkyl, Alkenyl, Aryl, Alkoxy, Alkylthio oder eine cyclische Aminogruppe oder
$R^3$ und $R^4$ vervollständigen zusammen einen ankondensierten, insbesondere carbocyclischen Ring

und wobei mindestens einer der Substituenten $R^1$, $R^2$, $R^3$ und $R^4$ einen die Diffusion erschwerenden Ballastrest mit 10–22 C-Atomen enthält.

Farbphotographisches Aufzeichnungsmaterial mit mindestens einer lichtempfindlichen Silberhalogenidemulsionsschicht und einer dieser zugeordneten Kombination aus einer nicht diffundierenden

reduzierbaren farbgebenden Verbindung, die im reduzierten Zustand unter den alkalischen Entwicklungsbedingungen einen diffundierenden Farbstoff oder Farbstoffvorläuferverbindungen freizusetzen vermag, und einer nicht diffundierenden Elektronendonor-Vorläuferverbindung (ED-Vorläuferverbindung), aus der unter den alkalischen Entwicklungsbedingungen eine nicht diffundierende Elektronendonor-Verbindung (ED-Verbindung) gebildet wird, die unter den alkalischen Entwicklungsbedingungen die nicht diffundierende farbgebende Verbindung zu reduzieren vermag, dadurch gekennzeichnet, daß das Aufzeichnungsmaterial als ED-Vorläuferverbindung eine Verbindung der folgenden allgemeinen Formel enthält:

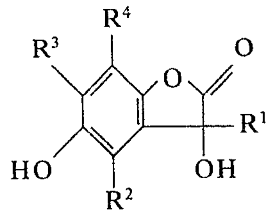

worin bedeuten

$R^1$ einen carbocyclischen oder heterocyclischen aromatischen Ring,
$R^2$, $R^3$, $R^4$ gleiche oder verschiedene Substituenten, und zwar Wasserstoff, Alkyl, Alkenyl, Aryl, Alkoxy, Alkylthio oder eine cyclische Aminogruppe oder
$R^3$ und $R^4$ vervollständigen zusammen einen ankondensierten, insbesondere carbocyclischen Ring

und wobei mindestens einer der Substituenten $R^1$, $R^2$, $R^3$ und $R^4$ einen die Diffusion erschwerenden Ballastrest mit 10—22 C-Atomen enthält.

4. Material nach Anspruch 3, dadurch gekennzeichnet, daß $R^1$ einen Phenylring bedeutet.

5. Material nach Anspruch 3, dadurch gekennzeichnet, daß die nicht diffundierende reduzierbare farbgebende Verbindung und die ED-Vorläuferverbindung jede in einem Ölbildner gelöst in heterodisperser Form enthalten sind.

6. Material nach Anspruch 5, dadurch gekennzeichnet, daß die nicht diffundierende reduzierbare farbgebende Verbindung und die ED-Vorläuferverbindung gemeinsam in einem Ölbildner gelöst in heterodisperser Form enthalten sind.

7. Material nach Anspruch 5 oder 6 dadurch gekennzeichnet, daß als Ölbildner Dialkylamide von Alkansäuren mit 8—22 C-Atomen enthalten sind.

8. Material nach Anspruch 7, dadurch gekennzeichnet, daß als Ölbildner Dialkylamide von Laurin- oder Palmitinsäure enthalten sind.

9. Verbindung der allgemeinen Formel

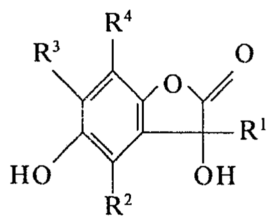

worin bedeuten

$R^1$ einen carbocyclischen oder heterocyclischen aromatischen Rest,
$R^2$, $R^3$, $R^4$ gleiche oder verschiedene Substituenten, und zwar Wasserstoff, Alkyl, Alkenyl, Aryl, Alkoxy, Alkylthio oder eine cyclische Aminogruppe oder
$R^3$ und $R^4$ vervollständigen zusammen einen ankondensierten, insbesondere carbocyclischen Ring

und wobei mindestens einer der Substituenten $R^1$, $R^2$, $R^3$ und $R^4$ einen die Diffusion erschwerenden Ballastrest mit 10—22 C-Atomen enthält.

10. Verbindung nach Anspruch 9, worin $R^1$ eine Phenylgruppe und $R^2$, $R^3$ und $R^4$ je eine Alkylgruppe bedeuten.

**Claims**

Colour photographic recording material having at least one light sensitive silver halide emulsion layer and, associated with this layer, a nondiffusible electron donor precursor compound (ED precursor compound) from which a non-diffusible electron donor compound (ED compound) is formed under the alkaline development conditions, characterised in that the recording material contains, as ED precursor compound, a 5-membered $\alpha$-lactone of hydroquinone whose lactone ring contains electron accepting substituents which at pH values of from 10 to 13 effect splitting up of the lactone ring with formation of an ED compound having a redox potential less that +0.225 V (determined at pH 0 against a normal calomel electrode), the splitting up of the lactone ring, measured in half life periods for the oxidative degradation of the lactone compound, determined in a heterodisperse mixture of a solution of

20 mg of the ED precursor compound and
5 mg of 1-phenyl-pyrazolidone-3 in
5 ml of n-butanol

as finely divided organic phase and a solution of

50 mg of potassium ferricyanide,
10 ml of water and
5 ml of a buffer solution

at room temperature, amounting to more than 10 minutes in a first measurement and less than 10 minutes in a second measurement if the first measurement is carried out using a buffer solution consisting of

3.092 g of boric acid ($H_3BO_3$),
3.728 g of potassium chloride,
0.852 g of sodium hydroxide
ad 50 ml of water

and the second measurement is carried out using a buffer solution consisting of

3.728 g of potassium chloride,
1.884 g of sodium hydroxide
ad 50 ml of water.

2. Colour photographic recording material having at least 1 light sensitive silver halide emulsion layer and, associated with this layer, a non-diffusible electron donor precursor compound (ED precursor compound) from which a non-diffusible electron donor compound (ED compound) having a high reduction capacity is formed under the alkaline development conditions, characterised in that the recording material contains, as ED precursor compound, a compound corresponding to the following general formula:

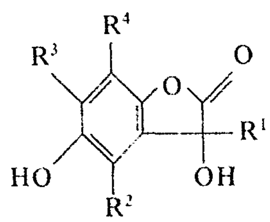

wherein

$R^1$ represents a corbocyclic or heterocyclic aromatic group,
$R^2$, $R^3$ and $R^4$ represent identical or different substituents, viz hydrogen, alkyl, alkenyl, aryl, alkoxy, alkylthio or a cyclic amino group or
$R^3$ and $R^4$ together complete a condensed, in particular a carbocyclic ring,

and at least one of the substituents $R^1$, $R^2$, $R^3$ and $R^4$ contains a ballast group having 10—22 carbon atoms which renders diffusion more difficult.

3. Colour photographic recording material having at least one light sensitive silver halide emulsion layer and, associated with this layer, a combination of a non-diffusible, reducible colour providing compound which in the reduced state is capable of releasing a diffusible dye precursor compounds under the alkaline development conditions and a non-diffusible, electron donor precursor compound (ED precursor compound) from which a non-diffusible electron donor compound (ED compound) is formed under the alkaline development conditions, which electron donor compound is capable of reducing the non-diffusible, colour providing compound under the alkaline development conditions, characterised in that the recording material contains, as ED precursor compound, a compound corresponding to the following general formula:

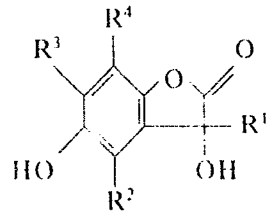

wherein

$R^1$ represents a carbocyclic or heterocyclic aromatic ring,
$R^2$, $R^3$ und $R^4$ represent identical or different substituents, viz hydrogen, alkyl, alkenyl, aryl, alkoxy, alkylthio or a cyclic amino group, or
$R^3$ and $R^4$ together complete a condensed, in particular a carbocyclic ring,

and at least one of the substituents, $R^1$, $R^2$, $R^3$ and $R^4$ contains a ballast group having 10–22 carbon atoms which renders diffusion more difficult.

4. Material according to Claim 3, characterised in that $R^1$ represents a phenyl ring.

5. Material according to Claim 3, characterised in that the non-diffusible, reducible, colour providing compound and the ED precursor compound are each contained in a heterodisperse form dissolved in an oil former.

6. Material according to Claim 5, characterised in that the non-diffusible, reducible, colour providing compound and the ED precursor compound are together contained in a heterodisperse form dissolved in an oil former.

7. Material according to Claim 5 or Claim 6, characterised in that it contains, as oil formers, dialkylamides of alkanoic acids having 8–22 carbon atoms.

8. Material according to Claim 7, characterised in that it contains, as oil formers, dialkylamides of lauric or palmitic acid.

9. Compound corresponding to the general formula:

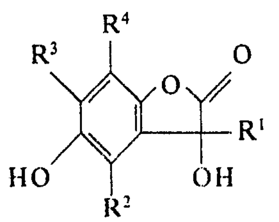

wherein

$R^1$ represents a carbocyclic or heterocyclic aromatic residue,
$R^2$, $R^3$ und $R^4$, which may be identical or different, represent hydrogen, alkyl, alkenyl, aryl, alkoxy, alkylthio or a cyclic amino group, or
$R^3$ and $R^4$ together complete a condensed, in particular a carbocyclic ring,

and at least one of the substituents, $R^1$, $R^2$, $R^3$ and $R^4$ contains a ballast group with 10–22 C atoms which renders diffusion more difficult.

10. Compound according to Claim 9, wherein $R^1$ represents a phenyl group and $R^2$, $R^3$ and $R^4$ each represent an alkyl group.

**Revendications**

1. Support d'enregistrement pour la photographie en couleurs comportant au moins une couche photosensible d'émulsion à l'halogénure d'argent laquelle est adjoint un composé précurseur de donneur d'électrons non diffusible (composé précurseur de DE) à partir duquel un composé donneur d'électrons (composé DE) non diffusible est formé dans les conditions alcalines de développement, caractérisé en ce que le support d'enregistrement contient comme composé précurseur de DE une $\alpha$-lactone à 5 chaînons de l'hydroquinone, dont le noyau de lactone contient des substituants attirant les électrons qui produisent, à des valeurs de pH comprises entre 10 et 13, une ouverture du noyau de la lactone avec formation d'un composé DE avec un potentiel d'oxydo-réduction de moins de +0,255 V (mesuré à un pH égal à 0 par rapport à une électrode normale au calomel), l'ouverture du noyau de lactone, mesurée en temps de demi-réaction pour la dégradation par oxydation de la lactone, étant effectuée dans un mélange en dispersion hétérogène formé d'une solution contenant

20 mg du composé précurseur de DE
5 mg de 1-phénylpyrazolidone-3 dans
5 ml de n-butanol

sous forme de phase organique finement dispersée et d'une solution de

50 mg de ferricyanure de potassium
10 ml d'eau et
5 ml d'une solution tampon

à la température ambiante, s'élevant dans une première mesure à plus de 10 minutes et dans une seconde mesure à moins de 10 minutes, lorsque la première mesure est effectuée en utilisant une solution tampon formée de

3,092 g d'acide borique ($H_3BO_3$)
3,728 g de chlorure de potassium
0,852 g d'hydroxyde de sodium
volume complété à 50 ml avec de l'eau

et la seconde mesure étant effectuée en utilisant une solution tampon formée de

3,728 g de chlorure de potassium
1,884 g d'hydroxyde de sodium
volume complété à 50 ml avec de l'eau.

2. Support d'enregistrement pour la photographie en couleurs comportant au moins une couche photosensible d'émulsion à l'halogénure d'argent à laquelle est adjoint un composé précurseur de donneur d'électrons non diffusible (composé précurseur de DE) à partir duquel un composé donneur d'électrons (composé DE) non diffusible à haut pouvoir réducteur est formé dans les conditions alcalines du développement, caractérisé en ce que le support d'enregistrement contient comme composé précurseur de DE un composé de formule générale:

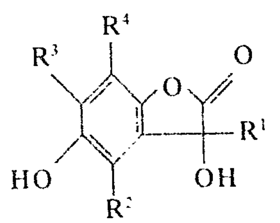

dans laquelle:

$R^1$ désigne un reste aromatique carbocyclique ou hétérocyclique;
$R^2$, $R^3$, $R^4$ sont des substituants égaux ou différents, à savoir hydrogène, alkyle, alcényle, aryle, alkoxy, alkylthio ou un groupe amino cyclique ou bien
$R^3$ et $R^4$ complètent ensemble un noyau condensé, notamment carbocyclique

et l'un au moins des substituants $R^1$, $R^2$, $R^3$ et $R^4$ contient un reste inerte ayant 10 à 22 atomes de carbone, rendant la diffusion plus difficile.

3. Support d'enregistrement pour la photographie en couleurs comportant au moins une couche photosensible d'émulsion à l'halogénure d'argent à laquelle est adjoint l'association formée d'un composé chromogène réductible non diffusible qui permet de libérer, à l'état réduit dans les conditions alcalines de développement, un colorant diffusible ou des composés précurseurs de colorants diffusibles, et d'un composé précurseur de donneur d'électrons non diffusible (composé précurseur de DE), à partir duquel est formé dans les conditions alcalines de développement un compose 1 donneur d'électrons (composé DE) non diffusible qui peut réduire dans les conditions alcalines de développement le composé chromogène non diffusible, caractérisé en ce que le support d'enregistrement contient comme composé précurseur de DE un composé de formule générale suivante:

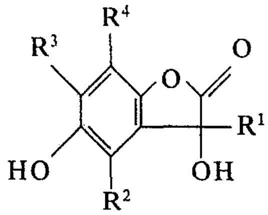

dans laquelle:

$R^1$ désigne un noyau aromatique carbocyclique ou hétérocyclique,
$R^2$, $R^3$, $R^4$ sont des substituants égaux ou différents, à savoir hydrogène, alkyle, alcényle, aryle, alkoxy, alkylthio ou un groupe amino cyclique ou bien
$R^3$ et $R^4$ complètent ensemble un noyau condensé, notamment carbocyclique

et l'un au moins des substituants $R^1$, $R^2$, $R^3$ et $R^4$ contient un reste inerte ayant 10 à 22 atomes de carbone, rendant la diffusion difficile.

4. Support suivant la revendication 3, caractérisé en ce que $R^1$ désigne un noyau phényle.

5. Support suivant la revendication 3, caractérisé en ce que le composé chromogène réductible non diffusible et le composé précurseur de DE sont contenus chacun sous une forme dispersée hétérogène en solution dans une substance oléifiante.

6. Support suivant la revendication 5, caractérisé en ce que le composé chromogène réductible non diffusible et le composé précurseur de DE sont contenus ensemble sous une forme dispersée hétérogène en solution dans une substance oléifiante.

7. Support suivant la revendication 5 ou 6, caractérisé en ce que des dialkylamides d'acides dérivés d'alcanes ayant 8 à 22 atomes de carbone sont contenus comme substances oléifiantes.

8. Support suivant la revendication 7, caractérisé en ce que des dialkylamides de l'acide laurique ou de l'acide palmitique sont contenus comme substances oléifiantes.

9. Composé de formule générale

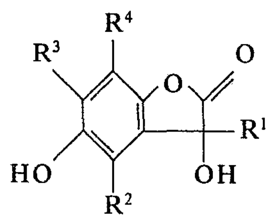

dans laquelle:

$R^1$ désigne un reste aromatique carbocyclique ou hétérocyclique,
$R^2$, $R^3$, $R^4$ sont des substituants égaux ou différents, à savoir hydrogène, alkyle, alcényle, aryle, alkoxy, alkylthio ou un groupe amino cyclique ou bien
$R^3$ et $R^4$ complètent ensemble un noyau condensé, notamment carbocyclique

et l'un au moins des substituants $R^1$, $R^2$, $R^3$ et $R^4$ contient un reste inerte ayant 10 à 22 atomes de carbone, rendant la diffusion plus difficile.

10. Composé suivant la revendication 9, dans lequel $R^1$ est un groupe phényle et $R^2$, $R^3$ et $R^4$ représentent chacun un groupe alkyle.